# EUROPEAN PATENT SPECIFICATION

(11) **EP 0 466 222 B1**
(45) Date of publication and mention of the grant of the patent: **08.09.1999**
(21) Application number: 91201436.2
(22) Date of filing: 10.06.1991
(51) Int. Cl.: C07K 14/415, C12N 15/29, C12N 5/10

(54) **Ribosome-inactivating proteins, inactive precursor forms thereof, a process for making and a method of using**
Ribosomen-inaktivierende Proteine, inaktive Vorläuferformen derselben, Verfahren zur Herstellung und eine Methode zur Nutzung
Protéines inactivant des ribosomes, formes de précurseurs inactivées de cela, un procédé de production et une méthode d'utilisation

(30) Priority: 11.06.1990 US 535636
(43) Date of publication of application: 15.01.1992
(73) Proprietor: Dow AgroSciences LLC, Indianapolis, Indiana 46268 (US)
(72) Inventor: Walsh, Terence A., Midland, Michigan 48640 (US); Hey, Timothy D., Midland, Michigan 48640 (US); Morgan, Alice E. R., Midland, Michigan 48640 (US)
(74) Representative: Smulders, Theodorus A.H.J., Ir.

(56) References cited:
- EP-A- 0 335 476
- GB-A- 2 216 891
- EUROPEAN JOURNAL OF BIOCHE- MISTRY, vol. 148, 1985, New York, Berlin F. I. LAMB et al. " Nucleotidesequence of cloned cDNA codingfor preproricin" pages 265-270
- CANCER SURVEYS, vol. 1, no. 3,1982, Oxford, England L. BARBIERI and F. STIRPE "Ribosome-inactivating pro- teins from plants: Properties and possible uses" pages 489-520
- W.H. Coleman and W.K. Roberts, Biochim. Biophys. Acta 1982, Vol. 696, pages 239-244

## Description

Ribosome-inactivating proteins (RIPs) are plant proteins that are capable of catalytically inactivating eukaryotic ribosomes and are consequently extremely potent inhibitors of eukaryotic protein synthesis. RIPs have been divided into two classes: type 1 and type 2 RIPs (see Barbieri and Stirpe (1982), Cancer Surveys, **1**:489-520). There is significant amino acid sequence homology between members of both type 1 and type 2 RIPs, and with the bacterial Shiga and Shiga-like toxins which also have the same mechanism of action (see Hovde *et al.* (1988), Proc. Natl. Acad. Sci. USA, **85**:2568-2572).

Type 2 RIPs consist of two polypeptides: an RIP (or A-chain) which is covalently attached via a disulfide bond to a lectin-like protein (or B-chain) with an affinity for cell surface carbohydrates. The B-chain binds to the cell surface and facilitates subsequent cellular internalization of the RIP A-chain moiety, which results in rapid inactivation of protein synthesis and cell death. Type 2 RIPs are therefore extremely potent cytotoxins and animal poisons, the best studied example of which is ricin.

In contrast type 1 RIPs, characterized to date, consist of a single polypeptide chain equivalent in activity to that of A-chain RIPs but lacking a covalently attached B-chain. Consequently, they are scarcely toxic to intact cells but retain their extreme potency against cell-free protein translation systems. Typical concentrations that inhibit cell-free protein translation by 50 percent (IC₅₀) are 0.5 to 10 ng/ml (0.16 to 33 pM). Until the discoveries detailed hereinbelow, reported type 1 RIPs were a remarkably homogeneous class of basic proteins with Mr values of about 30,000. Type 1 RIPs are found in a great variety of dicot and monocot plants and they may be ubiquitous. They are often abundant proteins in seeds, roots or latex for example. Their *in vivo* function is unclear but it has been proposed that they may be antiviral agents (see Stevens *et al.* (1981), Experientia, **37**:257-259) or antifungal agents (see Roberts and Seltrennikkoff (1986), Bioscience Reports, **6**:19-29).

To date, one article has discussed the presence of an inhibitor of animal cell-free protein synthesis in maize, as well as other cereal crops (see Coleman and Roberts (1982), Biochimica et Biophysica Acta, **696**:239-244). The preparation of the maize inhibitor was via ammonium sulfate precipitation and phosphocellulose column chromatography. It is generally believed that the inhibitor isolated from maize was pure. The reported molecular weight of the inhibitor was 23 kiloDaltons (kD), with a reported IC₅₀ of 50 to 100 ng/ml in an ascites cell-free protein synthesis assay.

Where the effect of RIPs on ribosomes has been examined, both type 1 and type 2 RIPs possess a unique and highly specific N-glycosidase activity which cleaves the glycosidic bond of adenine 4324 of the rat liver ribosomal 28S RNA (see Endo (1988), In:Immunotoxins, (ed.) Frankel).

Commercial interest in RIPs has primarily focused on their use in construction of therapeutic toxins targeted to specific cells such as tumor cells by attachment of a targeting polypeptide, most frequently a monoclonal antibody (see Immunotoxins (1988), *supra*). This mimics the binding functionality of the B-chain of type 2 RIPs but replaces their non-specific action with a highly selective ligand. Another recent potential use is in HIV therapy (see U.S. Patent 4,869,903 to Lifson *et al.*, (Genelabs Incorporated and The Regents of the University of California)).

However, while a maize-derived protein synthesis inhibitor, like protein synthesis inhibitors from other *Panicoideae* would appear to be useful for construction of cytotoxic conjugates, no artisan to date has reported to have successfully used a *Panicoideae* RIP. This is somewhat surprising in view of the success achieved with RIPs from non-*Panicoideae* plants, including cereals such as barley (see Lambert *et al.* (1988), In:Immunotoxins,, *supra*). In part, the lack of success to date by skilled artisans in successfully utilizing the maize RIP described by Coleman and Roberts may be attributed to the fact that the protein synthesis inhibitor was relatively uncharacterized.

There is interest in expressing recombinant RIP in commonly employed host eukaryotic cells, because of the capacity to provide correct post-translational processing. However, as RIPs effectively inhibit protein synthesis in eukaryotic cells, a predictable problem is that heterologous expression of an RIP will result in host cell death. Thus, eukaryotic cells are generally not used as recombinant host cells. Although eukaryotlc cells may be used in certain circumstances, the RIP must be constructed so as to be secreted prior to the cell experiencing toxicity (see EP 0 335 476 to Gelfand *et al.* (Cetus Corp.)). Therefore, prokaryotic host cells are generally used as hosts, notwithstanding disadvantages such as the inability to glycosylate and properly fold heterologously expressed proteins.

It is thus an object of the invention to provide a method of preparing inactive forms of RIPs, in which an inactive RIP may be expressed by eukaryotic host cells and then converted to an active form.

It is yet another object of the invention to provide the DNA sequence of the gene encoding at least one inactive form of RIP, as well as expression vehicles, host cells and cell cultures containing such DNA sequence.

Other objects and advantages of the present invention will become apparent from the teachings presented hereinafter.

It is to these objects to which the present invention is directed.

In a first aspect, the present invention is directed to a DNA isolate as identified in the claims encoding a homogeneous protein, termed a proRIP, wherein the protein is incapable of substantially inactivating eukaryotic ribosomes, but which can be converted into a protein, termed an αβ RIP, that is capable of substantially inactivating eukaryotic ribosomes, said proRIP having a removable, internal peptide linker sequence.

In a second aspect, the present invention is directed to a DNA isolate as identified in the claims encoding a homogeneous protein derived from *Panicoideae*, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes.

In a third aspect, the present invention is directed to a DNA isolate as identified in the claims encoding a homogeneous protein derived from *Panicoideae*, termed an αβ RIP, that is capable of substantially inactivating eukaryotic ribosomes, wherein the RIP has α and β fragments.

In a fourth aspect, the present invention is directed to a DNA isolate as identified in the claims encoding a homogeneous protein derived from maize, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes, said α fragment having an amino acid sequence effectively homologous to the following sequence: 
KRIVPKFTEIFPVEDANYPYSAFIASVRKDVIKHCTDHKGIFQPVLPPEKKVPELW
FYTELKTRTSSITLAIRMDNLYLVGFRTPGGVWWEFGKD
GDTHLLGDNPRWLGFGGRYQDLIGNKGLETVTMGRAEMTR
AVNDLAKKKK
 and said β fragment having an amino acid sequence effectively homologous to the following sequence:

AADPQADTKSKLVKLVVMVCEGLRFNTVSRTVDAGFNSQHGVTLTVTQGKQVQKWDRI
SKAAFEWADHPTAVIPDMQKLGIKDKNEAARIVAL
VKNQTTAAAATAASADNDDDEA.

In a fifth aspect, the present invention is directed to a method of converting a protein that is incapable of substantially inactivating eukaryotic ribosomes into a protein that is capable of substantially inactivating eukaryotic ribosomes, said method comprising the following steps:
a) providing a homogeneous protein derived from *Panicoideae* encoded by a DNA isolate as identified in the claims, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes; and
b) contacting the proRIP with a cleaving agent capable of deleting the linker to form a protein having α and β fragments, termed an αβ RIP, that is capable of substantially inactivating eukaryotic ribosomes.

In a sixth aspect, the present invention is directed to a conjugate comprising a targeting vehicle and a protein derived from *Panicoideae* encoded by a DNA isolate as identified in the claims, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes.

In a seventh aspect, the present invention is directed to a conjugate comprising a targeting vehicle and a protein derived from *Panicoideae* encoded by a DNA isolate as identified in the claims, termed an αβ RIP, that is capable of substantially inactivating eukaryotic ribosomes, wherein the αβ RIP has α and β fragments.

In a eighth aspect, the present invention is directed to a conjugate comprising a targeting vehicle and a protein derived from maize encoded by a DNA isolate as identified in the claims, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes, said α fragment having an amino acid sequence effectively homologous to the following sequence: 
KRIVPKFTEIFPVEDANYPYSAFIASVRKDVIKHCTDHKGIFQPVLPPEKKVPELW
FYTELKTRTSSITLAIRMDNLYLVGFRTPGGVWWEFGKD
GDTHLLGDNPRWLGFGGRYQDLIGNKGLETVTMGRAEMTR
AVNDLAKKKK
 and the β fragment having an amino acid sequence effectively homologous to the following sequence:

AADPQADTKSKLVKLVVMVCEGLRFNTVSRTVDAGFNSQHGVTLTVTQGKQVQKWDRI
SKAAFEWADHPTAVIPDMQKLGIKDKNEAARIVAL
VKNQTTAAAATAASADNDDDEA.

In a ninth aspect, the present invention is directed to DNA isolate as identified in the claims capable of encoding a protein, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes.

In a tenth aspect, the present invention is directed to a DNA isolate as identified in the claims encoding a protein, termed an αβ RIP, that is capable of substantially inactivating eukaryotic ribosomes, wherein the αβ RIP has α and β fragments.

In an eleventh aspect, the present invention is directed to a DNA isolate as identified in the claims encoding a protein derived from maize, termed a proRIP, wherein the proRIP has a removable, internal peptide linker sequence and is incapable of substantially inactivating eukaryotic ribosomes, but which upon removal of the linker can be converted into a protein having α and β fragments, termed an αβ RIP, that is capable of substantially inactivating eukaryotic ribosomes, said α fragment having an amino acid sequence effectively homologous to the following sequence: 
KRIVPKFTEIFPVEDANYPYSAFIASVRKDVIKHCTDHKGIFQPVLPPEKKVPELW
FYTELKTRTSSITLAIRMDNLYLVGFRTPGGVWWEFGKD
GDTHLLGDNPRWLGFGGRYQDLIGNKGLETVTMGRAEMTR
AVNDLAKKKK
 and said β fragment having an amino acid sequence effectively homologous to the following sequence:

AADPQADTKSKLVKLVVMVCEGLRFNTVSRTVDAGFNSQHGVTLTVTQGKQVQKWDRI
SKAAFEWADHPTAVIPDMQKLGIKDKNEAARIVAL
VKNQTTAAAATAASADNDDDEA.

In a twelfth aspect, the present invention is directed to a DNA isolate as identified in the claims encoding a fusion protein capable of substantially inactivating eukaryotic ribosomes, said protein having an amino acid sequence effectively homologous to the following sequence:

KRIVPKFTEIFPVEDANYPYSAFIASVRKDVIKHCTDHKGIFQPVLPPEKKVPELW
FYTELKTRTSSITLAIRMDNLYLVGFRTPGGVWWEFGKD
GDTHLLGDNPRWLGFGGRYQDLIGNKGLETVTMGRAEMTR
AVNDLAKKKKAADPQADTKSKLVKLVVMVCEGLRFNTVSRTVDAGFNSQHG
VTLTVTQGKQVQKWDRISKAAFEWADHPTAVIPDMQKLGIKDKN
EAARIVALVKNQTTAAAATAASADNDDDEA.

In other aspects, the invention is directed to expression vehicles capable of effecting the production of such aforementioned proteins in suitable host cells. It also includes the host cells and cell cultures which result from transformation with these expression vehicles.

A number of aspects of the present invention are further illustrated in the accompanying Drawings, in which:

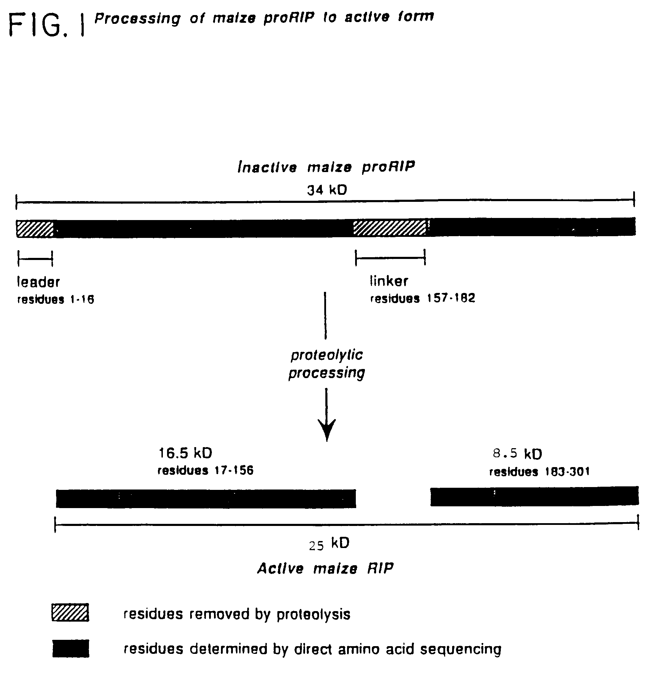

Figure 1 shows a schematic representation of the processing of maize proRIP to the active form.
Figure 2 shows the effect of active maize αβ RIP on mammalian cell-free protein synthesis.

The entire teachings of all references cited herein are incorporated by reference.

### Definitions

Nucleic acids, amino acids, peptides, protective groups, active groups and so on, when abbreviated, are abbreviated according to the IUPACIUB (Commission on Biological Nomenclature) or the practice in the fields concerned. The following are examples.

The single letter code for amino acids is set forth below:

| | | | |
|---|---|---|---|
| Glycine: | G | Phenylalanine: | F |
| Alanine: | A | Tyrosine: | Y |
| Valine: | V | Threonine: | T |
| Leucine: | L | Cysteine: | C |
| Isoleucine: | I | Methionine: | M |
| Serine: | S | Glutamic acid: | E |
| Aspartic acid: | D | Tryptophan: | W |
| Lysine: | K | Proline: | P |
| Arginine: | R | Asparagine: | N |
| Histidine: | H | Glutamine: | Q |
| Unknown | X | | |

The term "proRIP" means a precursor protein that contains a leader and linker and that is not capable of inactivating eukaryotic ribosomes.

The term "leader" refers to an N-terminal amino acid sequence of a proRIP that need not present in the mature, fully active form of the αβ RIP.

The term "linker" refers to an internal amino acid sequence within a proRIP, whereby the linker is of a length and contains residues effective to render the proRIP incapable of catalytically inhibiting translation of a eukaryotic ribosome.

The term "RIP" means a protein that is capable of inactivating eukaryotic ribosomes. The term "αβ RIP" means a RIP having an α fragment, which may or may not contain the leader, a β fragment and being capable of substantially inactivating eukaryotic ribosomes.

The term "IC₅₀" means the concentration of a protein necessary to inhibit protein synthesis by 50 percent in a cell-free protein synthesis assay.

The term "inhibiting amount" refers to the specific ability of RIPs to cause the death or injury of cells against which they are targeted.

The term "target cells" means those cells having ribosomes which the αβ RIP of the present invention is capable of inhibiting. The target cells may be present in living organisms or they may be preserved or maintained *in vitro*. The cells may be individual or associated to form an organ. Exemplary target cells include any eukaryotic cell (e.g., mammalian, insect, fungal and plant cells).

The term "targeting vehicle" means a carrier moiety containing a ligand capable of binding to a receptor of a specific cell or tissue.

"Gene" refers to the entire DNA portion involved in the synthesis of a protein. A gene embodies the structural or coding portion which begins at the 5' end from a translation start codon and extends to a stop codon at the 3' end. It also contains a promoter region, usually located 5' or upstream to the structural coding portion, which initiates and regulates the expression of a structural gene and a 3' nontranslated region downstream from the translated region.

"Expression" refers to a two-part process for the transcription and translation of a gene. The DNA defining the gene is transcribed into a precursor RNA, which is processed to its mature form, messenger RNA (mRNA). During translation, the cell's ribosomes, in conjunction with transfer RNA, translate the RNA "message" into proteins.

### Preferred Embodiments of the Invention

Surprisingly, it has been discovered that studied members of *Panicoideae* contain αβ RIP and proRIP. *Panicoideae* is a subfamily of *Gramineae* (order) and *Graminaceae* (family). The subfamily *Panicoideae* contains three tribes: *Maydeae* (e.g., *Tripsacum*, *Coix*, *Euchlaena* and *Zea*), *Andropogonea* (e.g., *Sorghum*) and *Paniceae*. For further taxonomic information, see Arber (1934), The *Gram**ineae*, A Study of Cereal, Bamboo and Grass, Cambridge University Press, p 410-411.

The present invention pertains to proteins which are derived from a plant within the subfamily *Panicoideae*. As taught herein, proteins obtained from various plants within the subfamily *Panicoideae* have shown antigenic cross reactivity (i.e., showing evidence of proRIP in *Panicoideae* as well as α and β fragments of an αβ RIP).

By "derived" from a plant within the subfamily *Panicoideae* means a protein that is effectively homologous, as defined below, with a proRIP or αβ RIP from *Panicoideae*, regardless of the manner in which the protein is produced. Given the present teachings it now becomes possible to prepare generally homogeneous proRIP and αβ RIP exclusive of irrelevant proteins and contaminants naturally associated therewith in the cellular environment or in extracellular fluids. For example, a substantially pure protein will show constant and reproducible characteristics within standard experimental deviations for parameters such as the following: molecular weight, chromatographic behavior, and such other parameters. The term, however, is not meant to exclude artificial or synthetic mixtures of a protein with other compounds. The term is not meant to exclude the presence of minor impurities which do not interfere with the biological activity of the protein and which may be present, for example, due to incomplete purification.

Both the proRIP and αβ RIP may be purified directly from mature and germinating seeds and developing kernels of plants within the subfamily. Generally, the purification of the *Panicoideae* αβ RIP and proRIP may be accomplished as follows.

Seeds or immature kernels of plants within the subfamily *Panicoideae* may be homogenized to disrupt the individual seeds or kernels. This can be accomplished by any type of commercially available homogenizer.

The *Panicoideae* proRIP and/or αβ RIP may be purified from the homogenization product by any appropriate protein purification technique. Exemplary techniques include gel filtration chromatographic techniques, such as conventional liquid chromatography, ion exchange chromatography, high performance liquid chromatography, and reverse phase chromatography.

Upon purification, the *Panicoideae* proRIP will have insignificant ribosome inactivating ability relative to its corresponding αβ RIP. For example, maize proRIP has an IC₅₀ of greater than 10 micrograms per milliliter (µg/ml) in a cell-free protein synthesis assay. The maize αβ RIP has an IC₅₀ of about 1 nanogram per milliliter (ng/ml) in a mammalian cell-free protein translation assay.

The maize proRIP has a molecular weight of about 34 kD, as determined by SDS-PAGE (see Laemmli (1970), *supra*), and will move as a single peak on ion exchange chromatography. Homogeneous maize αβ RIP will comprise two associated fragments, an α and β fragment, having molecular weights of approximately 16.5 kD and 8.5 kD, respectively (as determined by sodium dodecyl sulfate polyacrylamide-gel electrophoresis (SDS-PAGE) (see Laemmli ((1970), Nature, **22**:680-685). The homogeneous protein will exhibit two dissociated peaks on reverse phase chromatography, and a single associated peak on ion exchange chromatography. Polyclonal anti-sera against each fragment both crossreact with a polypeptide present in maize kernels having a molecular weight of about 34 kD as determined by SDS-PAGE. This demonstrates that the two fragments of the maize αβ RIP are in fact derived from a common precursor (i.e., the maize proRIP).

The maize proRIP amino acid sequence (as set forth in Table 1) contains four sequence subsegments:
(1) a leader sequence, from residues 1 to 16;
(2) an α fragment, from residues 17 to 161;
(3) an internal linker sequence, from residues 162 to 186; and
(4) a β fragment, from residues 187 to 301.

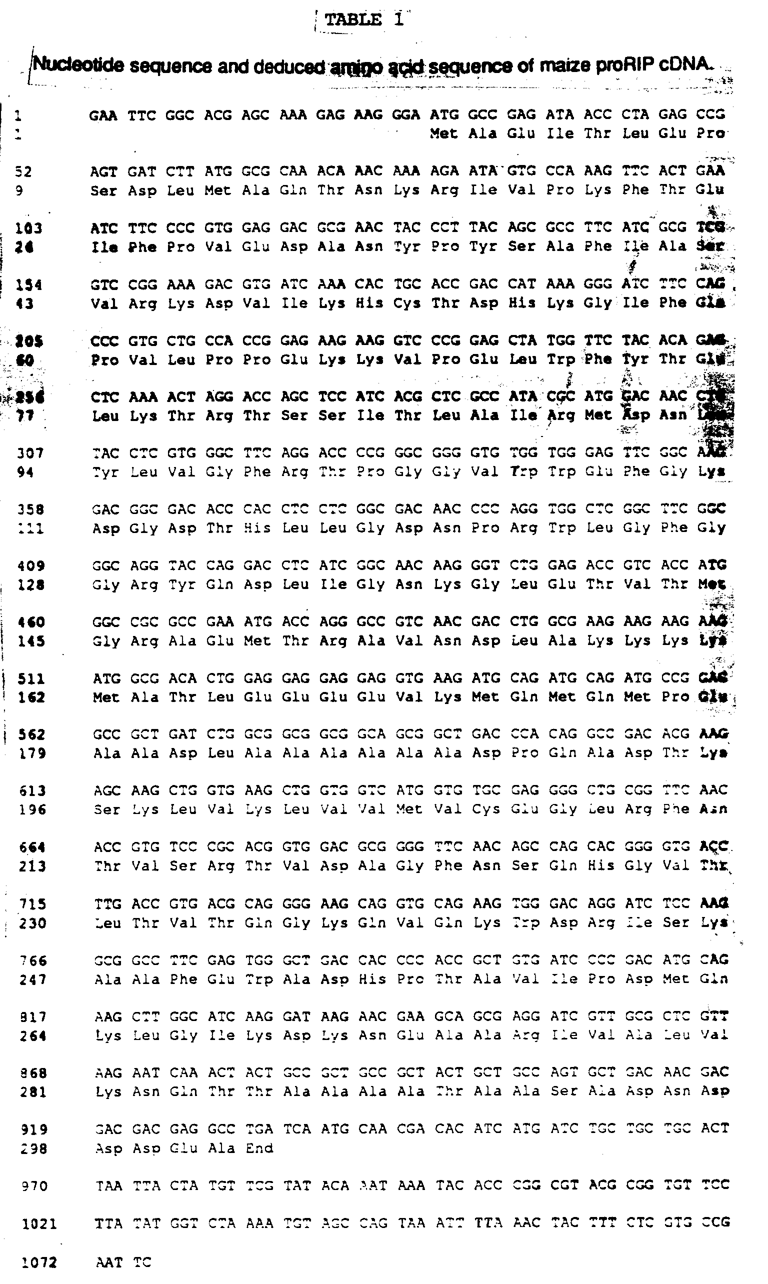

The net charges of these polypeptides are as follows: leader -3; α, +10; linker, -5; β, +1. Removal of the leader and linker results in a dramatic change in net charge from the maize proRIP (+3) to maize αβ RIP (+11). Additionally, the proRIP isolated from maize has an observed pI of 6.5 which agrees well with the value of 6.1 derived from the deduced amino acid sequence. The pI of the active maize αβ RIP is ≧9, compared to the calculated value from the deduced amino acid sequence of 9.06 (i.e., after deletion of the acidic leader, and linker sequences). Experimental data further suggests that the native maize αβ RIP has a few amino acids removed from the carboxy-terminal end. Thus, the maize αβ RIP has a basic pI, which is consistent with the pI of other RIPs.

A comparison of the maize proRIP with that of barley, a monocot , as set forth in Table 2 below. The upper sequence shows maize αβ RIP and the lower sequence barley RIP, as taught by Asano *et al.* (1986), Carlsberg Res. Commun., **51**:129. Identical residues are denoted by a solid line, conservative substitutions by a dotted line, and dashes indicate insertions to maximize homology. Residues are numbered on the left.

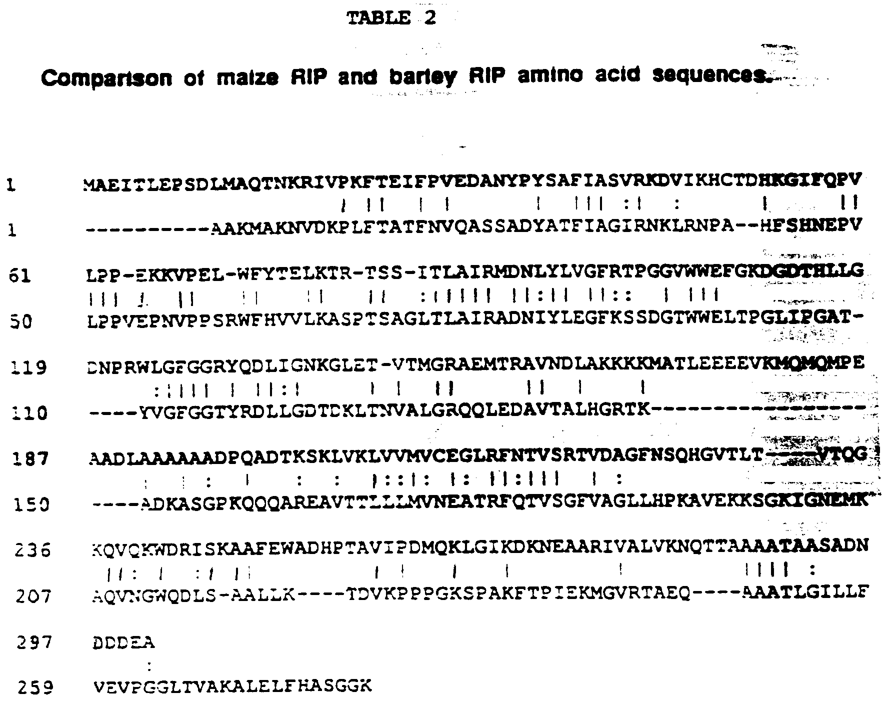

As set forth in in Table 2, there is an overall homology of 28 percent (34 percent including conservative substitutions) between the maize αβ RIP and barley RIP. However, the unique nature of the linker region of maize proRIP is clearly shown by the resulting gap that has been introduced in the published barley sequence to maintain homology. A lower, but significant, degree of homology is seen when the maize proRIP sequence is compared to that of ricin A-chain (as set forth in Table 3 below). The upper sequence is maize αβ RIP and the lower sequence is ricin A, as taught by Lamb *et al.* (1985), Eur. J. Biochem., **148**:265. Identical residues are denoted by a solid line and conservative substitutions by a dotted line, dashes indicate insertions to maximize homology. Residues are numbered on the left, the numbering of the ricin sequence corresponds to that of the mature protein.

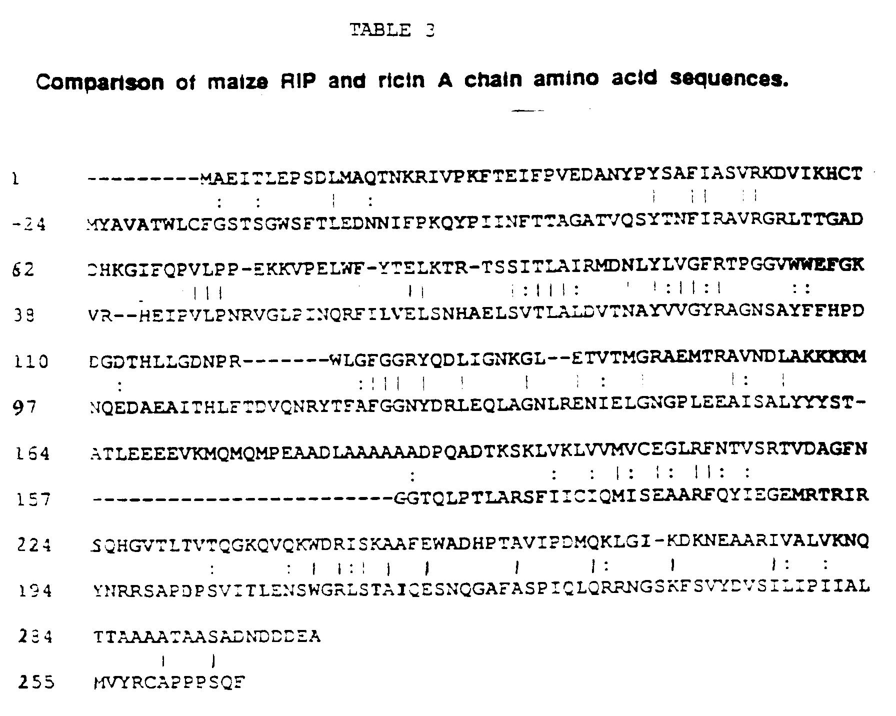

As set forth in Table 3, a gap was again introduced in the published ricin A sequence to maximize homology corresponding to the linker region of the maize proRIP.

Further comparison of the maize proRIP sequence with published full-length sequences of other non-*Panicoideae* RIPs indicate that there are four regions of significant homology between these proteins (as set forth in Tables 4a and 4b below).

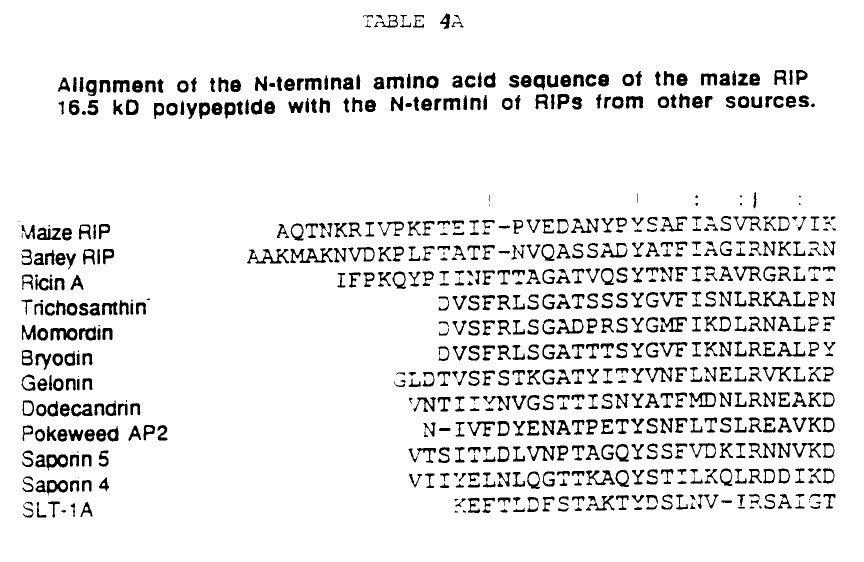

Table 4A shows the first region and the comparative alignment of the N-terminal amino acid sequence of the maize αβ RIP α fragment with the N-terminal sequences of RIPs from other sources. The sequences are taken from: barley (see Asano *et al.* (1986), *supra*); ricin A-chain (see Lamb *et al.* (1985), *supra*); dodecandrin (see Ready *et al.* (1985), Biochem. Biophys, Acta, **791**:314); pokeweed anti-viral protein 2 (AP2) (see Bjorn *et al.* (1985), Biochim. Biophys. Acta, **790**:154); Shiga-like toxin 1A (SLT-IA) (see Calderwood *et al.* (1987), Proc. Nat. Acad. Sci. USA, **84**:4364); and α-trichosanthin, momordins, bryodin, gelonin, dodeoandrin, pokeweed antiviral protein-2, saporin 5, and saporin 4 (see Montecucchi *et al.* (1989), Int. J. Peptide Res., **33**:263). Positions showing homology in four or more sequences are noted by solid lines (showing identical residues) or dotted lines (showing conservatively substituted residues).

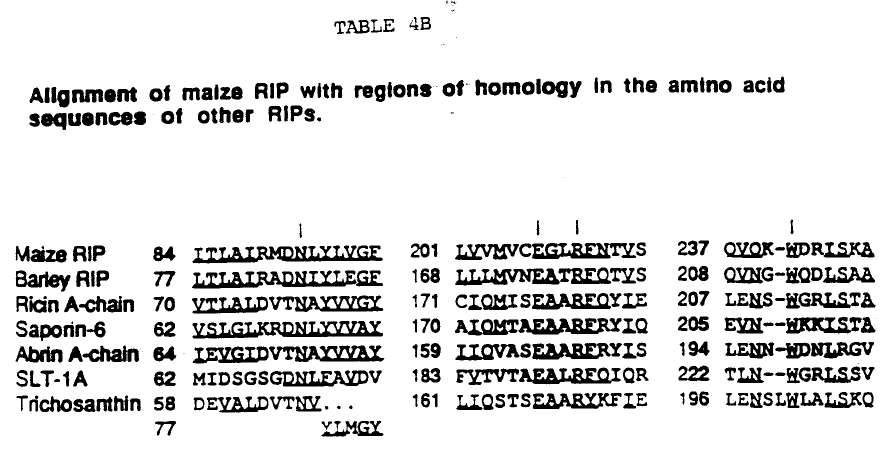

Table 4b shows that the other three regions are internally oriented. Table 4b specifically shows the alignment of maize αβ RIP with regions of homology in the amino acid sequences of other RIPs. The sequences are available from the following references: barley (see Asano *et al.* (1986), *supra* and Leah *et al.* (1991), J. Biol. Chem., **266**:1564-1573); ricin A-chain (see Lamb *et al.* (1985), *supra*); abrin A-chain (see Funatsu *et al.* (1988), Agric. Biol. Chem. **52**:1095); saporin-6 (see Benatti *et al.* (1989) Eur. J. Biochem., **183**:465); Shiga-like toxin 1A (SLT-1A) (see Calderwood *et al.* (1987), *supra*); and α-trichosanthin (see Xuejun and Jiahuai (1986), Nature, **321**:477; Chow *et al.* (1990), J. Biol. Chem., **265**:8670-8674 and Maragonore *et al.* (1987), J. Biol. Chem., **262**:11628-11633). Positions showing identity or conservative substitutions in four or more sequences are underlined, dashes indicate insertions to maximize homology. Vertical lines indicate residues that are conserved in all seven sequences. The starting amino acid of each sequence is indicated (note that trichosanthin contains an insertion sequence at residues 67 to 76).

The sequences and partial sequences of various additional Type I RIPs are set forth in the following articles: luffin-A (see Islam *et al.* (1990), Agric. Biol. Chem., **54**:2967-2978; mirabilis antiviral protein (see Habuka *et al.* (1989) J. Bio. Chem., **264**:6629-6637; trichokirin, (see Casellas *et al.* (1988), Eur. J. Biochem., **176**:581-588; momordins (see Barbieri *et al.* (1980), Biochem. J., 186:443-452; dianthins (see Reisbig and Bruland (1983), Arch. Biochem. Biophys., **224**:700-706; saporins (see Maras *et al.*, (1990), Biochem. Intl., **21**:831-838 and Lappi *et al.* (1985), Biochem. Biophys. Res Commun., **129**:934-942; and momorcochin-S (see Bolognesi *et al.* (1989), Biochim. Biophys. Acta, **993**:287-292.

Other Type I and Type II RIPs have also been purified to homogeniety and these include; momorcharins (see Yeung *et al.* (1986), Int. J. Peptide Res., **28**:518-524; tritins (see Roberts and Stewart (1979), Biochem., **18**:2615-2621); rye (see Coleman and Roberts (1982), Biochim. Biophys. Acta, **696**:239-244; agrostins and *Hura crepitans* (see Stirpe *et al.* (1983), Biochem. J., **216**:617-625; *Asparagus officianalis* (see Stirpe *et al.* (1983), Biochem. J., **216**:617-625; *Cucumis melo* (see Ferreras *et al.* (1989), Biochem Intl., **19**:201-207; *Cucurbitaceae* (see Ng *et al.*, Int. J. Biochem., **21**:1353-1358; *Petrocoptis* (see Ferreras *et al.*, Cell. Molec. Biol., **35**:89-95); volkensin-a (see Barbieri *et al.* (1984), FEBS Lett., **171**:277-279; viscumin-a (see Olsnes *et al.* (1982), J. Biol. Chem., **257**:13263-123270; modeccin-a (see Gasperi-Campani (1978), Biochem. J., **174**:491-496); *Momordia charantia* lectin-a (see Lin *et al.* (1978), Linn. Toxicon., **16**:653-660); *Phorandendron californicum* lectin-a (see Franz *et al.* (1989), FEBS lett., **248**:115-118).

Proteins from the following other plants have also been shown to possess ribosome inactivating activity: *Stellarea holostea*, *Lychnis flos-cuculi*, *Hordeum murinum*, *Aegylops geniculata*, *Euphorbia serrata*, *Capsella bursapastoris*, *Muscari comosum* (see Merino *et al.* (1990), J. Exp Botany, **41**:67-70); and proteins from *Croton tiglium* and *Jatropha curcas* (see Stirpe *et al.* (1976), Biochem J., **156**:1-6).

When the internal linker sequence of the proRIP is removed, the αβ RIP has significant ribosome inactivating activity. The activity has been found to be significant regardless of whether the leader sequence has been removed (e.g., by recombinant methods). However, the proRIP is most active when the leader sequence is also removed and probably when C-terminus residues are also removed. In nature, it is thought that the linker is cleaved by endogenous proteases released by germinating seeds. Significantly, it has been discovered that the linker may also be cleaved *in vitro* by certain proteases, e.g., papain, to yield active maize αβ RIP from the precursor. While not intended to be bound by theory, it is thought that papain likely mimics the effect of endogenous endoproteinases released during germination.

It appears that, after removal of the internal linker, the two fragments of the processed polypeptide are held together by noncovalent forces. That is, the association of the two polypeptide chains does not depend upon interchain disulfide bonds or the formation of a peptide bond between the fragments.

Although not intended to be bound by theory, it is believed that the linker forms an external loop with exposed amino acid residues that are susceptible to proteolysis. Support for this suggestion comes from the alignment of the amino acid sequence of the maize proRIP with that of ricin A chain, the three dimensional structure of which is known (see Montfort *et al*, (1987), J. Biol. Chem., _**262**:5398). The Glu 177, Arg 180, Asn 209 and Trp 211 of ricin A have been implicated in the active site region of the molecule (see Robertus (1988), In:Immunotoxins, *supra*).

Based on this alignment, homologous residues of maize αβ RIP can be positioned within the three dimensional structure of ricin A chain. The superimposed structures indicate that the C-terminal lysine of the α fragment (at residue 162) is in corresponding alignment with an externally positioned threonine (at residue 156) of the ricin A-chain. Also, the N-terminal alanine of the β fragment (at residue 189) is in corresponding alignment with an externally positioned glycine (at residue 157) of the ricin A-chain.

The present invention is intended to include the construction of αβ RIP and proRIP forms of any RIP. As set forth in Tables 4a and 4b, RIPs for which a full-length sequence has been determined contain regions with significant homology. Similarly the N-terminal sequence similarities in an even greater number of RIPs have been compared (set forth in Tables 4a and 4b). It is likely that these regions have particular effect upon the function of the respective RIP.

An RIP having known amino acid sequences may now be altered into inactive, proRIP forms by the insertion of a linker. Based on the information deduced from the maize system set forth herein, it now becomes possible to engineer inactive forms of any RIP having a three dimensional structure similar to the three dimensional structure of ricin A chain. Cleavage of the linker will result in an αβ RIP not heretofore found in nature.

The art has discussed the methodology for modifying the three dimensional structure of proteins (see, for example, Van Brunt (1986), Biotechnology, **4**:277-283). The first step involves selecting plausible sites on the RIP between which the linker may be inserted. One of those sites is the exposed amino acid residues surrounding residue 156 of ricin A-chain or its equivalent in other RIP sequences. Thus, the present invention is intended to encompass the insertion of a peptide linker in those sequences, provided that the insertion of the linker substantially reduces the ribosome inactivating ability of the RIP. By "substantially reduce" is meant that the insertion of a cleavable linker into an active RIP lowers the IC₅₀ value of the resultant protein by at least 10 fold, preferably 100-fold, and more preferably 1000-fold.

As stated previously, ricin A-chain has been shown to have sequence homology to many single chain RIPs. The RIPs set forth in Tables 4a and 4b are intended for exemplification purposes only. RIPs characterized in the future that meet the above criteria are also considered to be a part of this invention.

Generally, the linker may be of a length, may be of an amino acid sequence, and may be internally positioned so as to substantially reduce the ribosome inactivating activity of the RIP.

Obviously, since the *Panicoideae* linker(s) is the only known RIP linker found in nature, it is expected that such an amino acid sequence will logically be a primary candidate for insertion into other RIPs. However the present invention is intended to encompass linkers having effectively homologous sequences to a selected maize linker. The factors to be considered in synthetically preparing effectively homologous linkers for αβ RIPs generally are the same as set forth above for selecting effectively homologous linkers for a selected maize linker.

Any of a variety of procedures may be used to clone proRIP-encoding gene sequence. One method for cloning the proRIP gene sequence entails determining the amino acid sequence of the proRIP molecule. To accomplish this task proRIP or αβ RIP protein may be purified (as described above), and analyzed to determine the amino acid sequence of the proRIP or αβ RIP. Any method capable of elucidating such a sequence can be employed, however, Edman degradation is preferred. The use of automated sequenators is especially preferred.

It is possible to synthesize *in vitro* the proRIP and αβ RIP from their constituent amino acids. A suitable technique includes the solid phase method (see Merrifield (1963), J. Amer. Chem. Soc., **85**:2149-2154; and Solid Phase Peptide Synthesis (1969), (eds.) Stewart and Young). Automated synthesizers are also available.

The peptides thus prepared may be isolated and purified by procedures well known in the art (see Current Protocols in Molecular Biology (1989), (eds.) Ausebel, *et al.*, Vol. 1) and Sambrook *et al.* (1989), Molecular Cloning: A Laboratory Manual).

Using the amino acid sequence information, the DNA sequences capable of encoding them are examined in order to clone the gene encoding the proRIP. Because the genetic code is degenerate, more than one codon may be used to encode a particular amino acid.

Although it is possible to determine the entire amino acid sequence of the proRIP or αβ RIP, it is preferable to determine the sequence of peptide fragments of the molecule, and to use such sequence data to prepare oligonucleotide probes which can be used to isolate the entire proRIP gene sequence. The proRIP peptide fragments can be obtained by incubating the intact molecule with cyanogen bromide, or with proteases such as papain, chymotrypsin or trypsin.

Using the genetic code one or more different oligonucleotides can be identified. The probability that a particular oligonucleotide will, in fact, constitute the actual proRIP encoding sequence can be estimated by considering abnormal base pairing relationships and the frequency with which a particular codon is actually used (to encode a particular amino acid) in eukaryotic cells. Using these rules, a single oligonucleotide, or a set of oligonucleotides, that contains a theoretical "most probable" nucleotide sequence capable of encoding the proRIP or αβ RIP peptide sequences may be identified.

The oligonucleotide, or set of oligonucleotides, containing the theoretical "most probable" sequence capable of encoding the proRIP gene fragments may be used to identify the sequence of a complementary oligonucleotide, or set of oligonucleotides, which is capable of hybridizing to the "most probable" sequence, or set of sequences. An oligonucleotide containing such a complementary sequence can be employed as a probe to identify and isolate the toxin gene (see Sambrook *et al.* (1989), *supra*).

By hybridizing an oligonuleotide having a sequence complementary to the "most probable" gene sequence, one obtains a DNA molecule (or set of DNA molecules), capable of functioning as a probe to identify and isolate the proRIP gene.

The present invention also relates to DNA sequences that encode recombinant proRIP and αβ RIP. The recombinantly-produced proRIP and αβ RIP share the following properties with the proRIP and αβ RIP isolated from nature and characterized according to the teachings herein: (1) portions of the amino acid sequence deduced from the nucleotide sequence are equivalent to amino acid sequences obtained directly from nature; (2) the polypeptide is recognized by anti-RIP antibodies; (3) the molecular weight of the proRIP and αβ RIP polypeptides encoded corresponds with the naturally occurring proteins; (4) each proRIP protein is convertable to an αβ RIP; and (5) each proRIP and αβ RIP protein exhibits relatively equivalent ribosome inactivating activity.

The process for genetically engineering the proRIP or αβ RIP according to the invention is facilitated through the cloning of genetic sequences which are capable of encoding the proRIP or αβ RIP, or effectively homologous variants thereof as discussed below, and through the expression of such genetic sequences. As used herein, the term "genetic sequences" is intended to refer to a nucleic acid molecule (preferably DNA). Genetic sequences which are capable of encoding the toxin may be derived from a variety of sources. These sources include genomic DNA, cDNA, synthetic DNA, and combinations thereof.

Cells containing the desired sequence may be isolated, and genomic DNA fragmented by one or more restriction enzymes. The genomic DNA may or may not include naturally-occurring introns. The genomic DNA digested with selected restriction endonucleases yields fragments containing varying numbers of base pairs (bp).

Specifically comprehended as part of this invention are genomic DNA sequences encoding allelic variant forms of the proRIP gene which may include naturally occurring introns. The allelic gene may be derived using a hybridization probe made from the DNA or RNA of the proRIP gene as well as its flanking regions. "Flanking regions" are meant to include those DNA sequences 5' and 3' of the proRIP encoding sequences.

The DNA isolate encoding the proRIP gene may also be obtained from a cDNA library. The mRNA may be isolated from a suitable source employing standard techniques of RNA isolation, and the use of oligo-dT cellulose chromatography to enrich for poly-A mRNA. A cDNA library is then prepared from the mixture of mRNA using a suitable primer, preferably a nucleic acid sequence which is characteristic of the desired cDNA. A single stranded DNA copy of the mRNA is produced using the enzyme reverse transcriptase. From the single stranded cDNA copy of the mRNA, a double-stranded cDNA molecule may be synthesized using either reverse transcriptase or DNA polymerase.

It is also possible to use primers to amplify the DNA from cells of appropriately prepared seeds and immature kernels by the polymerase chain reaction (PCR). PCR involves exponentially amplifying DNA *in vitro* using sequence specified oligonucleotides. (see Mullis *et al.* (1987), Meth. Enz., **155**:335-350; Horton *et al.* (1989), Gene, **77**:61; and PCR Technology: Principles and Applications for DNA Amplification, (ed.) Erlich (1989).

The DNA encoding the proRIP or αβ RIP may be chemically synthesized by manual procedures, e.g., the phosphotriester and phosphodiester methods (see Caruthers (1983), In:Methodology of DNA and RNA, (ed.) Weissman); or automated procedures e.g., using diethylphosphoramidites are used as starting materials (see Beaucage *et al.* (1981), Tetrahedron Letters, **22**:1859-1962). The DNA may be constructed by standard techniques of annealing and ligating fragments or by other methods.

Thereafter, the desired sequences may be isolated and purified by procedures well known in the art (see Current Protocols in Molecular Biology (1989), *supra*) and Sambrook *et al.* (1989), Molecular Cloning: A Laboratory Manual.

The nucleotide sequence of the maize proRIP cDNA and the deduced amino acid sequence of such the corresponding maize proRIP is set forth in Table 1.

One need not be confined to the amino acid sequences of proRIP and αβ RIP found in nature. Thus, it is possible to selectively produce both proRIP and αβ RIP via the application of recombinant DNA technology. This in turn allows the production of sufficient quality and quantity of material to create novel forms of the protein unimpeded by the restriction necessarily inherent in the isolation methods involving production and extraction of the protein from natural sources.

Recombinant procedures make possible the production of effectively homologous proteins possessing part or all of the primary structural conformation and/or one or more of the biological properties of the αβ RIP. For purposes of this invention, an amino acid sequence is effectively homologous to a second amino acid sequence if at least 70 percent, preferably at least 80 percent, and most preferably at least 90 percent of the active portions of the amino acid sequence are identical and retains its intended function. Thus more importantly and critical to the definition, an effectively homologous sequence to the αβ RIP retains the capacity to interact with and inactivate eukaryotic ribosomes. The effectively homologous sequence to the proRIP must retain the capacity to be converted into an αβ RIP. That is, the effectively homologous proRIP must have a linker sequence which, when cleaved, will yield a biologically functional αβ RIP.

General categories of potentially-equivalent amino acids are set forth below, wherein, amino acids within a group may be substituted for other amino acids in that group: (1) glutamic acid and aspartic acid; (2) lysine, arginine and histidine; (3) hydrophobic amino acids such as alanine, valine, leucine and isoleucine; (4) asparagine and glutamine; (5) threonine and serine; (6) phenylalanine, tyrosine and tryptophan; and (7) glycine and alanine..

It is envisioned that, compared with changes to the α and β fragments, more significant changes may be made to the proRIP in the leader and linker regions. That is, since the leader and linker sequences are to be cleaved, the length and amino acid residues in their sequences may better be tolerated and considered insignificant, because it will not alter the functionality of the final product.

Further, the linker sequence of the proRIP need not be limited to the sequence set forth in Table 1, above. For example, the length of the linker may be modified, provided that (1) the linker is cleavable, and (2) upon cleavage of the linker the resultant protein has an IC₅₀ value that is at least about 10 times lower than the IC₅₀ value of the protein containing the linker.

Primary criteria for selecting an effectively homologous linker include altering the net charge of the αβ RIP (e.g., more acidic); creating a conformational shift in the protein or providing steric hindrance to the active site of the protein.

As noted previously, the maize αβ RIP, like other RIPs, is basic. However, the maize proRIP has a slightly acidic pI. Thus, it is preferred that any effectively homologous linker selected for the maize proRIP will be acidic.

The linker should be of a length which, while capable of altering the three-dimensional structure of the protein, when cleaved will permit the protein to retain most of the three dimensional features of the active αβ RIP molecule.

To ensure that the linker is cleavable it is generally required that the conformation of the proRIP be such that the linker cleavage sites are readily accessible to a selected cleavage agent.

It is also envisioned that at least one restriction enzyme site may be engineered into the genetic sequence encoding an RIP, allowing DNA sequences encoding various polypeptide linkers to be inserted into the gene and tested for their ability to create an inactive, yet activatable RIP.

Most commonly, cleavage will be effected outside of the replicative environment, for example, following harvest of microbial culture. Thus, when genetically modifying the proRIP, it may be preferable, in some instances, that the internal linker domain of the proRIP be retained, or altered so as to mimic the manner in which a natural, inactive proRIP liberates its active α and β fragments.

Any chemical or enzymatic method which recognizes a specific sequence or structure and causes an appropriate cleavage at a selected site may be utilized for the present invention. For example, it may be desirable to design carboxy termini and amino termini of the linker sequences that are subject to cleavage with selected agents. Exemplary such sequences Pro-Xxx-Gly-Pro (where Xxx is unspecified) which is selectively cleaved by collagenase; Ile-Glu-Gly-Arg which is selectively cleaved by Factor Xa; and Gly-Pro-Arg which is selectively cleaved by thrombin (see Nilsson *et al.* (1988), In: Advances in Gene Technology; Protein Engineering and Production, (ed.) Brew *et al.*).

A chemical or enzymatic method may not be suitable if its cleavage site occurs within the active amino acid sequences of the α and β fragments. That is, cleavage within the native amino acid sequence of the α and β fragments will generally have a greater likelihood of deleteriously affecting the enzymatic activity of the αβ RIP. It is possible to select a specific cleavage sequence of only one amino acid residue so long as that residue does is not accessible in the amino acid sequences of the α and β fragments. It is preferred, however, to utilize a specific cleavage sequence which contains two or more amino acid residues, i.e., an extended specific cleavage sequence. This type of sequence takes advantage of the extended active sites of various enzymes. Additionally, by utilizing an extended specific cleavage sequence, it is highly probable that cleavage will only occur at the desired site and not at other sites within the protein.

The cleavage techniques discussed here are by way of example and are but representative of the many variants which will occur to the skilled artisan in light of the specification.

In some instances it may prove desirable to effect cleavage of the proRIP within the cell. For example, an expression vehicle with appropriate promoters may be provided with a DNA sequence coding for enzymes which convert the proRIP to the active form, operating in tandem with the other DNA sequence coding expression of the proRIP.

Further, as is well known, protein sequences may be modified by post-translation processing such as becoming associated with other molecules, for example, glycosides, lipids, or such inorganic ions as phosphate. The ionization status will also vary depending on the pH of the medium or the pH at which crystallization or precipitation of the isolated form occurs. Further, the presence of air may cause oxidation of labile groups, such as -SH. Thus, included within the definition of the proRIP and αβ RIP, and fragments, thereof are all such modifications of a particular primary structure, e.g., both glycosylated and non-glycosylated forms, neutral forms, acidic and basic salts, lipid or other associated peptide forms, side chain alterations due to oxidation or derivatization, and any other such modifications of an amino acid sequence which would be encoded by the same genetic codon sequence.

Exemplary techniques for nucleotide replacement include the addition, deletion or substitution of various nucleotides, provided that the proper reading frame is maintained. Exemplary techniques include using polynucleotide-mediated, site-directed mutagenesis, i.e., using a single strand as a template for extension of the oligonucleotide to produce a strand containing the mutation (see Zoller *et al.* (1982), Nuc. Acids Res., **10**:6487-6500; Norris *et al.* (1983), Nuc. Acids Res., **11**:5103-5112; Zoller *et al.* (1984), DNA, **3**:479-488; and Kramer *et al.* (1982), Nuc. Acids Res., **10**:6475-6485) and by using PCR, i.e., exponentially amplifying DNA *in vitro* using sequence specified oligonucleotides to incorporate selected changes (see PCR Technology: Principles and Applications for DNA Amplification, Erlich, (ed.) (1989); and Horton *et al.*, *supra*).

By appropriate choice of restriction sites, the desired DNA fragment may be positioned in a biologically functional vector which may contain appropriate control sequences not present in the selected DNA fragment. By "biologically functional" is meant that the vector provides for replication and/or expression in an appropriate host, either by maintenance as an extrachromosomal element or by integration into the host genome. A large number of vectors are available or can be readily prepared, and are well known to skilled artisans.

In general, vectors containing the appropriate promoters, which can be used by the host organism for expression of its own protein, also contain control sequences, ribosome binding sites, and transcription termination sites. Generally, the replicon and control sequences which are derived from species compatible with the host cell are used in connection with these hosts.

Finally, the vectors should desirably have a marker gene that is capable of providing a phenotypical property which allows for identification of host cells containing the vector.

When expressing the αβ RIP, the DNA fragments encoding the α fragment and the β fragment may be inserted into separate vectors, or into the same vector. Specifically, when the α and β fragments are contained in separate vectors, the host cells may be transformed via either cotransformation or targeted transformation techniques.

Construction of suitable vectors containing the desired coding and control sequences may be produced as follows.

Restriction endonucleases may be used as a means for inserting the DNA fragments containing the proRIP gene or αβ RIP genes into an appropriate expression vehicle. Exemplary restriction enzymes include *Aat* II, *Bam* HI, *Eco* RI, *Hind* III, *Nde* I, *Spe* I, *Xba* I, *Sac* I, *Bgl* II, *Pst* I, *Sal* I and *Pvu* II.

Cleavage is performed by treating the expression vehicle with a restriction enzyme(s). In general, 10 µg vector or DNA fragments is used with 10 units of enzyme in 100 µl of buffer solution. Endonuclease digestion will normally be carried out at temperatures ranging from 37 degrees Centigrade (37°C) to 65°C, at a pH of 7 to 9. Appropriate buffers and substrate amounts for particular restriction enzymes are specified by the manufacturers. Time for the reaction will be from 1 to 18 hours.

After the restriction enzyme digestion is complete, protein may be removed by standard techniques (e.g., extraction with phenol and chloroform). The nucleic acid may be then recovered from the aqueous fraction by standard techniques.

The desired fragment is then purified from the digest. Suitable purification techniques include gel electrophoresis or sucrose gradient centrifugation. The vector and foreign DNA fragments may then be ligated with DNA ligase.

An appropriately buffered medium containing the DNA fragments, DNA ligase, and appropriate cofactors is employed. The temperature employed will be between 4°C to 25°C. When DNA segments hydrogen bond, the DNA ligase will be able to introduce a covalent bond between the two segments. The time employed for the annealing will vary with the temperature employed, the nature of the salt solution, as well as the nature of the sticky ends or cohesive termini. Generally, the time for ligation may be from 5 to 18 hours (see Sambrook *et al.* (1989), *supra*).

Thereafter, the vector constructions may be used to transform an appropriate host cell. Suitable host cells include cells derived from unicellular as well as multicellular organisms which are capable of being grown in cultures or by fermentation.

Various unicellular microorganisms can be used for both cloning and expression. Prokaryotes include members of the Enterobacteriaceae, such as strains of *Escherichia coli*, and *Salmonella*; *Bacillaceae*, such as *Bacillus subtilis*; *Pneumococcus*, *Streptococcus*, and *Haemophilus influenzae*.

In addition to prokaryotes, eukaryotic cells may be employed. As previously stated, eukaryotic cells have not heretofore been used as recombinant host cells for RIPs. By providing inactive forms of RIPs, the present invention provides skilled artisans with the flexibility to use eukaryotic cells as recombinant hosts. By transforming eukaryotic cells with the proRIP gene, the protein may be expressed at high levels without being toxic to the host cell. Since the protein is lacking in bioactivity pending extra-cellular cleavage, the effect is to enhance the biosafety of the procedure. The proRIP may then be selectively chemically or enzymatically converted to the desired αβ RIP.

Exemplary eukaryotic microbes include yeast. *Saccharomyces cerevisae*, or common baker's yeast, is the most commonly used among eukaryotic microorganisms, although a number of other hosts are commonly available.

In addition to eukaryotic microbes, cultures of cells derived from multicellular organisms may also be used as hosts. Examples of useful host mammalian cell lines are Sp2/0, VERO and HeLa cells, Chinese hamster ovary (CHO) cell lines, and W138, BHK, COS-7 and MDCK cell lines.

Other suitable hosts and expression systems are the baculovirus systems maintained in cultured insect cells, e.g., from *Spodoptera frugiperda*.

Finally, cells from and portions of higher plants have been found useful as recombinant hosts, and appropriate control sequences are available for expression in these systems. Suitable plant cells include cells derived from, or seedlings of, tobacco, petunia, tomato, potato, rice, maize and the like.

The expression vehicle may be inserted into the host cell by any suitable method. Conventional technologies for introducing biological material into living cells include electroporation (see Shigekawa and Dower (1988), Biotechniques, **6**:742; Miller, *et al.* (1988), Proc. Natl. Acad. Sci. USA, **85**:856-860; and Powell, *et al* (1988), Appl. Environ. Microbiol., **54**:655-660); direct DNA uptake mechanisms (see Mandel and Higa (1972), J. Mol. Biol., **53**:159-162; Dityatkin, *et al.* (1972), Biochimica et Biophysica Acta, **281**:319-323; Wigler, *et al.* (1979), Cell, **16**:77; and Uchimiya, *et al.* (1982), In: Proc. 5th Intl. Cong. Plant Tissue and Cell Culture, A. Fujiwara (ed.), Jap. Assoc. for Plant Tissue Culture, Tokyo, pp. 507-508); fusion mechanisms (see Uchidaz, *et al.* (1980). In:Introduction of Macromolecules Into Viable Mammalian Cells, Baserga *et al.* (eds.) Wistar Symposium Series, **1**:169-185); infectious agents (see Fraley, *et al.* (1986), CRC Crit. Rev. Plant Sci., **4**:1-46; and Anderson (1984), Science, **226**:401-409); microinjection mechanisms (see Crossway, *et al.* (1986), Mol. Gen. Genet., **202**:179-185); and high velocity projectile mechanisms (see EPO 0 405 696 to Miller, Schuchardt, Skokut and Gould, (The Dow Chemical Company). The appropriate procedure may be chosen in accordance with the plant species used.

Generally after transformation, the host cells may be grown for about 48 hours to allow for expression of marker genes. The cells are then placed in selective medium and/or screenable media, where untransformed cells are distinguished from transformed cells, either by death or a biochemical property.

The transformed cells are grown under conditions appropriate to the production of the desired protein, and assayed for expression thereof. Exemplary assay techniques include enzyme-linked immunosorbent assay, radioimmunoassay, or fluorescence-activated cell sorter analysis, immunohistochemistry and the like.

Selected positive cultures are subcloned in order to isolate pure transformed colonies. A suitable technique for obtaining subclones is via the limiting dilution method.

### Uses

Essentially all of the uses that the prior art has envisioned for RIPs are intended for the novel αβ RIP and proRIP set forth herein (see Immunotoxins (1988), Frankel (ed.); and U.S. Patent 4,869,903 to Lifson *et al.* (Genelabs Incorporated and the Regents of the University of California)).

By providing inactive precursor forms of the αβ RIP, it is now possible to provide protein synthesis inhibitors with uses in practical and improved ways not before possible. The inactive form of the αβ RIP offers the additional advantage, over active RIPs, of not being active until removal of the linker sequence. Although the RIP is not toxic to the majority of mammalian cells it is known that RIP may be made specifically cytotoxic by attachment to a targeting vehicle which is capable of binding to and into target cells.

Exemplary targeting vehicles include any peptide hormone, growth factor, or other polypeptide cell recognition protein for which a specific receptor exists. A few examples include: antibodies and antibody fragments, lectins, insulin, glucagon, endorphins, growth hormone, melanocyte-stimulating hormone, transferrin, bombesin, low density lipoprotein, luteinizing hormone and asialoglycoprotein that bind selectively to target cells (see Immunotoxins (1988), *supra*). It is well established that conjugates which contain RIP exhibit maximal cytotoxicity only when the RIP moiety is released from the targeting vehicle.

Since the αβ RIP and proRIP does not contain a reactive sulfhydryl group, it may be necessary to modify the proteins using chemical crosslinking reagents in order to link such proRIP and αβ RIP to targeting vehicles.

Conjugates of a monoclonal antibody and the αβ RIP and proRIP may be made using a variety of bifunctional protein coupling agents. General examples of such reagents are N-succinimidyl-3-(2-pyridyldithio)propionate, 2-iminothiolane, bifunctional derivatives of imidoesters such as dimethyl adipimidate, active esters such as disuccinimidyl suberate, aldehydes such as glutaraldehyde, bis-azido compounds such as bis(p-diazoniumbenzoyl)ethylenediamine, diisocyanates such as toluene 2,6-diisocyanate, and bis-active fluorine compounds such as 1,5-difluoro-2,4-dinitrobenzene (see, for example, WO 86/05098).

In addition, recombinant DNA methodologies may be employed to construct a cytotoxic fusion protein. For a general discussion of chimeric toxins (see, for example, Pastan and FitzGerald (1989), The Journal of Biological Chemistry, **264**:15157-15160; and U.S. Patent 4,892,827 to Pastan *et al.* (The United States of America as represented by the Department of Health and Human Services)). These references teach modified *Pseudomonas* exotoxins which comprise a deletion in a receptor binding domain to form a fusion protein capable of rendering the modified toxin less toxic to selected cells. These references teach DNA sequences encoding the human alpha transforming growth factor (α-TGF) or human interleukin fused to toxin genes.

### Examples

The present invention is illustrated in further detail by the following examples. The examples are for the purposes of illustration only, and are not to be construed as limiting the scope of the present invention. All temperatures not otherwise indicated are Centigrade. All parts and percentages are by weight unless otherwise specifically noted.

### Example 1: Isolation of Maize αβ RIP

All steps were performed at 4°C, except for high performance liquid chromatography (HPLC) which was performed at room temperature. Five hundred grams (500 g) of finely ground mature maize kernels were extracted for at least 2 hour and up to 24 hours (hr) with 1500 ml, 25 mM sodium phosphate, pH 7.2 (PB) + 50 mM sodium chloride. After the extract was strained through several layers of cheesecloth, the protein precipitating between 55 percent and 85 percent ammonium sulfate was collected and redissolved in PB, then dialyzed overnight against the same buffer. The solution was clarified by centrifugation and applied to a 2.5 x 10 centimeter (cm) DE-52 cellulose column equilibrated with PB. The protein passing straight through the column was collected and applied to a Mono S 10/10 column (Pharmacia LKB Biotechnology, Piscataway, NJ) equilibrated with PS, and eluted with a linear gradient of 0 to 200 mM sodium chloride in PB over 90 min at 2 milliliter/ minute (ml/min). Alternatively, the protein can be precipitated with 85 percent ammonium sulfate and dialyzed overnight before applying to the Mono S 10/10 column.

Fractions containing ribosome inactivating protein activity (as measured by rabbit reticulocyte protein synthesis assay, described above) were pooled and concentrated to 0.5 ml in Centricon-10 devices (Amicon, Danvers, MA), and applied to a Superose 12 column equilibrated in PB (Pharmacia LKB Biotechnology) at a flow-rate of 0.4 ml/min. Fractions containing ribosome inactivating protein activity (as measured by a rabbit reticulocyte protein synthesis assay, described above) (the first major peak) were pooled. At this stage, the αβ RIP was usually quite pure as identified by SDS-PAGE (see Laemmli (1970), *supra*). If necessary, further purification can be achieved by applying the protein to a Mono S 5/5 column (Pharmacia LKB Biotechnology) equilibrated with PB and eluted at 1 ml/min with 0 to 50 mM sodium chloride in PB over 5 min, then 50 to 200 mM sodium chloride in PB over 25 min.

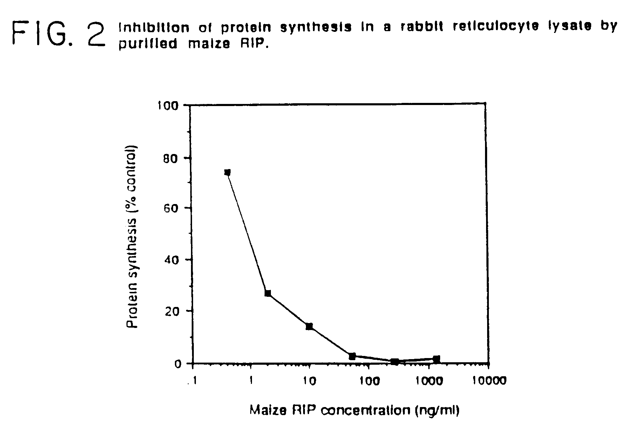

Results from a typical purification are presented in Table 5. The effect of purified maize αβ RIP on mammalian protein synthesis is shown in Figure 2.

**Table 5**

| **Purification of maize RIP from mature kernels** | | | | | |
|---|---|---|---|---|---|
| Step | Protein (mg) | Total units¹ x 10⁶ | Yield (%) | Fold Purification | IC₅₀ (ng/ml) |
| Crude extract | 6816 | 384 | 100 | 1.0 | 323 |
| 85% Ammonium sulfate | 1010 | 115 | 30 | 2.0 | 161 |
| post-DE52 treatment | 428 | 144 | 38 | 5.9 | 54 |
| Mono S 10/10 pool | 10.2 | 58 | 15 | 102 | 3.2 |
| Superose 12 pool | 1.8 | 33 | 8.6 | 327 | 0.99 |
| Mono S 5/5 pool | 1.32 | 32.4 | 8.4 | 436 | 0.74 |

| | | | | | |
|---|---|---|---|---|---|
| 1. One unit of activity is the amount of protein required to produce 50% inhibition in the rabbit reticulocyte lysate protein synthesis assay. | | | | | |

### A. Rabbit Reticulocyte Cell-Free Protein Synthesis Assay

The inhibitory activity of the maize αβ RIP toward mammalian protein synthesis was measured in a rabbit reticulocyte lysate system following the procedures of Pelham and Jackson (see (1976), Eur. J. Biochem., **67**:247-256).

A mix of the following reagents was prepared (2.5 milliliter (ml) total volume): 125 microliter (µl) 200 mM Tris-HCl, pH 7.6 + 40 mM magnesium acetate + 1.6 M potassium chloride; 12.5 µl 3 mM hemin hydrochloride in 50 percent ethylene glycol; 1.0 ml untreated rabbit reticulocyte lysate (Promega, Madison, WI); 1.0 ml H₂O; 62.5 µl amino acid mix; 125 µl 20 mM ATP + 4 mM GTP; 125 µl 200 mM creatine phosphate; 50 µl 2.5 mg/ml creatine phosphokinase in 50 percent ethylene glycol. The amino acid mix contained 50 µM of each amino acid except glycine (100 µM), arginine, isoleucine, methionine and tryptophan (10 µM each) and contained no leucine. All stock solutions were previously adjusted to pH 7.5 prior to addition.

Five microliters (5 µl) of appropriate dilutions of samples to be assayed were placed in the wells of a 96-well plate and 50 µl of the mix added. After 10 minutes, 50 nanoCuries (nCi) ¹⁴C-leucine in 10 µl was added to each well. After a further 10 minutes (min.), the reaction was quenched with 10 µl 1.5 M potassium hydroxide and incubated for 45 min. Twenty-five microliters (25 µl) of each sample was then pipetted onto individual 2.1 cm Whatman 3 MM paper disks (Whatman, Clifton, NJ) and after drying for 2 to 3 min, the disks were washed successively by swirling in a flask with 250 ml 10 percent trichloroacetic acid, 250 ml 5 percent trichloroacetic acid (twice), 125 ml ethanol, 250 ml 1:1 ethanol/acetone, and 125 ml acetone. After drying, the filters were placed in vials with 10 ml scintillation cocktail and counted.

### B. Antisera and Western blot analysis:

The α and β polypeptide bands were cut from 3 millimeter (mm) SDS-PAGE gels after brief staining with Coomassie blue and were electroeluted using an electroelution device (Bio-Rad, Richmond CA) according to the manufacturer's directions. The polypeptide preparations were then used to immunize rabbits to yield polyclonal anti-sera (prepared by RIBI Immunochem, Montana).

Western blots from Phastgels™ reagent (Pharmacia LKB Biotechnology) were performed by removing the gel from the plastic backing and then electroblotting onto Immobilon paper (Millipore Corporation, Bedford, MA). Blots were developed using the maize αβ RIP primary antiserum at 1:2000 dilution and alkaline phosphatase-conjugated goat anti-rabbit secondary antibody (Bio-Rad), according to the manufacturer's instructions.

### Example 2: Isolation of Maize proRIP

The polyclonal antisera against the α and β fragments were used to identify a common 34 kD precursor polypeptide in crude extracts of maize kernels (maize proRIP). The presence of the maize proRIP was monitored during subsequent purification by Western blot analysis as set forth above. All steps of the purification were performed at 4°C, except for HPLC which was performed at room temperature.

Two hundred fifty grams (250 g) of immature maize kernels were homogenized in 600 ml 25 mM sodium phosphate, pH 7.2 (PB) + 5 µg/ml antipapain. After the extract was strained through several layers of cheesecloth, the protein precipitating between 45 and 80 percent ammonium sulfate was collected and redissolved in 15 ml PB, then passed over a 2.5 x 15 cm Sephadex G-25 column (Pharmacia LKB) equilibrated in PB. Fractions containing protein were pooled and diluted to ∼60 ml with water. The solution was applied to a Q-Sepharose (fast-flow) column packed in a 10/10 FPLC column (Pharmacia LKB Biotechnology) equilibrated with PB, and eluted with a 0 to 300 mM NaCl gradient at 2 ml/min over 75 min. Fractions containing the 34 kD precursor were pooled and concentrated by a Centriprep 10 device (Amicon) to 1.5 ml. This was diluted four-fold with water and applied to a Mono Q 5/5 column (Pharmacia LKB Biotechnology) equilibrated in PB. The column was eluted with a 0 to 250 mM NaCl gradient over 60 min. Fractions containing the 34 kD polypeptide were pooled, concentrated to 0.5 ml and applied to a Superose 12 column (Pharmacia LKB Biotechnology) equilibrated in PB. The major peak from this column contained the 34 kD maize RIP precursor and appropriate fractions were pooled and stored at -20°C.

### Example 3: PAGE Analysis of Maize αβ RIP and proRIP

SDS-PAGE was performed with a Phastsystem™ reagent (Pharmacia LKB Biotechnology) using 20 percent Phastgels™ reagent and following the manufacturer's instructions. Native PAGE was performed at pH 4.2 as described in the Phastsystem™ reagent application file no. 300, method 1 (Pharmacia LKB Biotechnology).

SDS-PAGE of highly purified, active maize αβ RIP exhibited two polypeptides an α fragment (16.5 kD) and a β fragment (8.5 kD) under both reducing and non-reducing conditions. A single band was seen in native PAGE analysis of purified, active maize αβ RIP. The minimal Mr value of the associated, native maize αβ RIP was therefore 25 kD.

By SDS-PAGE, highly purified maize proRIP migrated with a value of 34 kD.

### Example 4: In vitro activation of Maize proRIP by Papain

A purified sample of proRIP was incubated at 0.5 mg/ml with papain, a plant thiol protease, at 0.01 mg/ml in sodium acetate buffer, pH 6 containing 2 mM dithiothreitol. After 1 to 2 hours at room temperature, the 34 kD proRIP was digested to a stable product exhibiting a polypeptide pattern almost identical to that of native, active maize αβ RIP. There was a concomitant increase in ribosome inactivating activity in the incubation; the undigested proRIP had no ribosome inactivating activity up to 2 µg/ml, whereas papain-treated proRIP had an IC₅₀ of <80 ng/ml. In contrast trypsin had no effect on maize proRIP.

### Example 5: Chemically-determined amino acid sequences

### A. N-Terminal Amino Acid Sequences of Maize αβ RIP α fragment and β

A sample of maize αβ RIP was electrophoresed by the method of Laemmli (1970), *supra*) in 1.5 mm thick gels and the gel electroblotted onto Immobilon PVDF paper (Millipore) using a Transphor™ apparatus (Pharmacia LKB Biotechnology). The paper was stained briefly with Coomassie blue and the bands corresponding to the 16.5 and 8.5 kD polypeptides were cut out. These were N-terminal sequenced directly from the PVDF paper using an 470A gas phase sequencer (Applied Biosystems, Foster City, CA). The following data was obtained (bracketed residues denote lower confidence assignments):

N-Terminal sequence of α fragment:
K R I V P K I T E I F P V E D A N Y P V S A F I A [G] V X K D V I

An additional minor species (∼20 percent of the total species) had an N-Terminal sequence of:
A Q T N K[L]I V P K

N-Terminal sequence of β fragment:
A A D P Q A D T K S X L V K L V V M S/C E G L X F N T V S

### B. α fragment C-Terminal Amino Acid Sequence

The carboxy-terminal amino acid sequence of the α maize αβ RIP a fragment was determined using sequencing grade carboxypeptidase P from *Penicillium japonicum* (Boehringer Mannheim, Indianapolis, IN). A sample of 16.5 kD polypeptide was purified by reverse-phase HPLC using a Vydac 5µ C4 4.6 x 30 mm RP column. The column was equilibrated with 0.1 percent trifluoroacetic acid (TFA), and eluted with 0 to 40 percent of 0.1 percent TFA + 80 percent acetonitrile over 8 min, then 40 to 60 percent of 0.1 percent TFA + 80 percent acetonitrile over 20 min. The β fragment eluted after 21.9 min. and the α fragment eluted after 23.3 min.

A lyophilized sample of the α fragment was dissolved in 20 mM sodium acetate, pH 5.8 + 4 M urea. The digestion mix contained the following in 0.1 ml: 1.6 µg carboxypeptidase P, 66 µg β fragment, 0.12 M sodium acetate pH 4.2, 0.8 M urea. After 1, 5, 15, 60, 120 and 480 min, duplicate 8 µl aliquots from the digestion were added to 8 µl 0.4 M sodium borate, pH 10.5 and frozen on dry ice.

Amino acid analysis was performed essentially as described by Jones (1986), In: Methods of Protein Microcharacterization (ed.) J.E. Shively. The following sequence is obtained: NH₂-Asp-Leu-Ala-(Lys)n-COOH, where n = 2-4. This was the carboxy terminus of the α polypeptide, therefore this and the N-terminus sequence of the β fragment define the linker region contained in the precursor (see amino acid sequence derived from cDNA in Table 1).

### C. N-Terminal Amino Acid Sequence of Maize proRIP

No N-Terminal sequence data was obtained from a sample of the 34 kD maize proRIP indicating that this polypeptide is N-terminal blocked.

### Example 6: Isolation and Characterization of cDNA for Maize proRIP

### A. Isolation

Immature kernels from field grown Pioneer hybrid 3737 were harvested, shelled from the cob, and stored at -20°C. Ten grams (10 g) of kernels were frozen in liquid nitrogen for several minutes then ground to a powder in a Waring blender. The powder was suspended in 20 ml of ice cold TENS buffer (10 mM Tris pH 7.4, 1 mM EDTA, 0.5 percent SDS, 0.3 M NaCl) and extracted immediately with an equal volume of phenol-chloroform-isoamyl alcohol (25:24:1) saturated with TENS buffer. The aqueous phase was collected and extracted three more times with fresh phenol mixtures.

Nucleic acids were precipitated from the aqueous phase by adjusting it to 0.3 M sodium acetate pH 5.5 and adding 2.5 volumes of 100 percent ethanol. Nucleic acids were collected by centrifugation and suspended directly in 1 ml phenol-chloroform-isoamyl alcohol plus 1 ml TENS and extracted by vortexing. The nucleic acid was precipitated from the aqueous phase by ethanol precipitation as above. The precipitate was collected by centrifugation and resuspended in TE buffer (10 mM Tris pH 7.4, 1 mM EDTA). Single strand nucleic acid was precipitated by adjusting the solution to 2M LiCl and incubating for 4 to 12 hours at 4°C. Centrifugation yielded a pellet which consisted of between 2.2 to 2.5 mg of total RNA.

Poly(A)-containing RNA was enriched from the total RNA sample by using Hybond mAP™ mRNA purification affinity paper (Amersham Corporation, Arlington Heights IL). The supplier's protocol was followed. Typically 5 to 10 µg of poly(A) enriched RNA were recovered per milligram of total RNA.

Five micrograms (5 µg) of poly(A) enriched RNA were converted into double stranded cDNA using a cDNA Synthesis™ kit (Pharmacia LKB Biotechnology). The cDNA was ligated into the cloning vector Lambda gt11 (Stratagene Inc., La Jolla CA) following the supplier's instructions. Packaging of the ligated vector-insert mixture was done with the Gigapack plus packaging extract (Stratagene, Inc.) again following the supplier's protocol.

The PicoBlue Immunodetection™ kit (Stratagene, Inc.) was used to screen the Lambda gt11 maize kernel cDNA library using rabbit polyclonal antisera raised against the maize proRIP, as described above.

Positive clones were purified to homogeneity and the cDNA inserts characterized by *Eco* RI restriction enzyme analysis. One of the largest *Eco* RI generated cDNA inserts (about 1,100 bp) was ligated into the *Eco* RI site of plasmid pUC19 (Bethesda Research Labs, Gaithersberg, MD). Clones carrying the proRIP cDNA insert in both orientations were identified by restriction digestion and used for large scale plasmid purification.

### B. Sequencing the maize proRIP cDNA

The nucleotide sequence of the proRIP cDNA (set forth in Table 1) was determined by dideoxy chain termination sequencing using the Sequenase™ DNA sequencing kit (United States Biochemical Corp., Cleveland Ohio). The double stranded pUC19-RIP was used as template following the manufacturer's instructions. The first round of sequencing was initiated by the M13/pUC forward sequencing primer (Bethesda Research Labs ). Subsequent primers were derived from the sequenced maize proRIP cDNA. Both strands of the cDNA were fully sequenced at least once.

The open reading frame encoding the αβ RIP protein was confirmed by comparing the cDNA deduced amino acid sequence (set forth in Table 1) to the chemically determined protein sequence data.

### Example 7: Expression of Maize proRIP and Derivatives in Escherichia coli

Various genetic derivatives of maize proRIP may be expressed in *E.coli* and tested for ribosome inactivating activity. A summary of several constructions and their properties is given below.

A. R34 (set forth in Table 6 below) represents the intact recombinant proRIP gene which encodes a protein of Mr 33,327 and as expected is not a potent inhibitor of protein synthesis. Upon papain treatment it is processed into two associated polypeptides (of approximately 17 + 9 kD) by SDS Phastgel™ analysis) with very potent ribosome inactivating activity. N34 represents the native proRIP as isolated from nature.

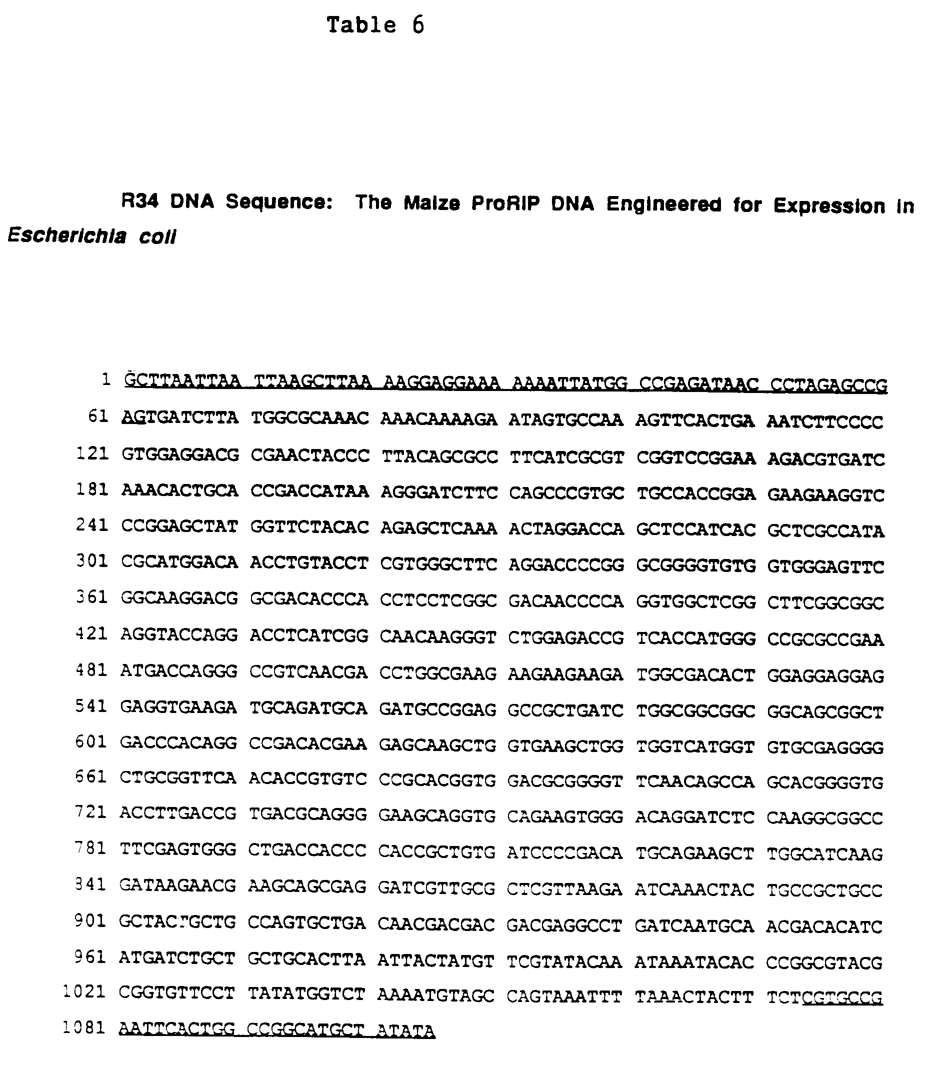

Expression of the recombinant maize proRIP in *E. coli* was accomplished by engineering the cDNA via PCR amplification. A 5' primer was synthesized which contained termination codons in all three reading frames to stop translation of vector-encoded proteins upstream of the maize proRIP cDNA. The primer also contained a Shine-Dalgarno sequence several base pairs upstream of an ATG start codon followed by 23 bases which were homologous to the maize proRIP cDNA. The 3' primer spans the 3' cDNA end-pUC19 junction (the primers were shown by the underlined regions set forth in Table 6). PCR amplification of the cDNA in pUC19 using the GeneAmp™ kit (Perkin Elmer-Cetus, Norwalk, CT) yielded a predominant amplification product of approximately 1100 base pairs, as expected.

The engineered, amplified product, was purified from an agarose gel and ligated into the filled-in *Hind* III site of the expression vector pGEMEX-1 (Promega Corp., Madison, WI) to give plasmid pGR, set forth below.

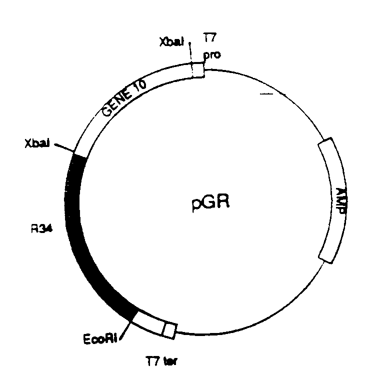

This was transformed into *E.coli* DH5α (Bethesda Research Labs). Plasmids containing the maize proRIP cDNA were isolated by colony hybridization (see Sambrook *et al.*, *supra*) with a 5' maize proRIP cDNA probe and characterized. Those containing the cDNA probe for the maize proRIP in the correct orientation were tested for expression. Plasmids were transformed into competent *E. coli* JM109(DE3) (Promega Corp., Madison, WI), transformed cells were grown in 15 ml cultures under ampicillin selection to an optical density at 600 nm of 0.4 to 1.0. Isopropylthio-β-galactoside (IPTG) was added to 1.3 mM to induce the production of recombinant proRIP and the cultures were grown an additional 4 hours at 37°C. The cells were collected by centrifugation and stored as a pellet at -20°C.

The protein produced from the maize proRIP cDNA was analyzed by lysing the induced cells in TE containing 1 mg/ml lysozyme 37°C for 15 min. The lysate was fractionated into a crude supernatant and pellet by microcentrifugation. The fractions were analyzed by SDS-PAGE using 20 percent Phastgels™ reagent (Pharmacia LKB Biotechnology). Coomassie blue staining and Western blot analysis of the gels with anti-maize proRIP sera identified a 34 kD band which was greatly increased upon induction of the cells with IPTG. Cells not carrying the plasmid or containing the plasmid with the maize proRIP cDNA in the inverted orientation did not contain this 34 kD immuno-reactive band. The majority of the recombinant maize proRIP was contained in the cellular pellet suggesting the material was insoluble under these conditions.

To test if R34 could acquire the folding pattern of N34 the pellet fraction of an induced culture was dissolved in 6M guanidine HCl and allowed to denature at room temperature for 3 hours. The material was then diluted 200-fold into ice cold TE and incubated at 4°C overnight to allow refolding of the denatured R34. The diluted material was then concentrated by a Centricon 10 device (Amicon). To test whether refolded R34 could undergo the correct proteolytic processing to the fragmented form of the maize proRIP, the material was treated with 10 µg/ml papain for various times, and samples were analyzed by SDS-Phastgel and Western blot analysis. The R34 material was processed to a stable mixture of two immuno-reactive bands which comigrate with N34 papain-processed material indicating the correct proteolytic processing had occurred.

In an effort to simplify purification of the R34 polypeptide from induced lysates, the gene 10 coding region of the pGEMEX-1 vector was removed by cutting the maize proRIP gene-containing plasmid (pGR) with *Xba* I and gel purifying the vector/proRIP DNA away from the gene 10 encoding DNA. Recircularization of pGR, now minus the gene 10 coding region, resulted in a plasmid called pGR1 set forth below.

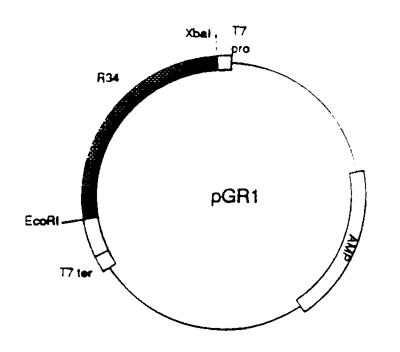

The plasmid pGR1 was transformed into JM109(DE3) cells and tested for production of R34 following induction with IPTG. As with pGR, large amounts of R34 were identified in cellular lysates both by Western blot and Coomassie blue staining. Unlike pGR, R34 produced from pGR1 was soluble and fractionated in the supernatant of lysed cells. This soluble material was treated with papain at 10 µg/ml and the R34 produced from pGR1 was cleaved to products which comigrate with N34, papain-cleaved product. The papain-treated material inhibited translation of reticulocyte lysates at significantly higher dilutions than the untreated material, indicating that the soluble R34 was processed to an active form.

B. R34-DL represents the proRIP without the linker. The sequences encoding α and β were joined directly without intervening linker DNA, i.e., nucleotides A-520 to A-594 are deleted. The R34-DL gene encoded a 30.6 kD protein which was a potent inhibitor of protein synthesis. Treatment of R34-DL with papain resulted in a 28 kD polypeptide with increased ribosome inactivating activity over the untreated molecule.

Confirmation that removal of the linker from maize proRIP activated the molecule was obtained independently through genetic engineering. The 75 bp linker encoding region of R34 (A-520 to A-594 inclusive, Table 6) was deleted using PCR amplification. The new construction R34-DL joined directly, in frame, the DNA encoding both the α and β fragments.

In the pGEMEX-1 system the R34-DL gene directed the synthesis of a polypeptide approximately 30.6 kD, which was recognized by antisera specific for the maize proRIP. At high dilution, *E.coli* lysates containing R34-DL protein were potent inhibitors of protein synthesis in rabbit reticulocyte lysates, in marked contrast to *E. coli* lysates containing the R34 polypeptide.

These genetic deletion data confirm that removal of the linker served to activate the R34 (proRIP) molecule. This experiment also demonstrated that covalent linkage of the α and the β polypeptide fragments resulted in an active αβ RIP. The maize proRIP did not require a break in the polypeptide backbone for enzymatic activity, removal of the linker region was sufficient to confer potent ribosome inactivating activity.

In addition, when R34-DL lysates were treated with papain a slight decrease in the molecular weight of R34-DL protein is noted (from 30.6 kD to approximately 28 kD). The R34-DL polypeptide remained intact, that is, it was not cleaved to the characteristic maize αβ RIP α and β fragments. Associated with this small change in molecular weight was an increase in protein synthesis inhibition in the *E.coli* lysates. These data indicated that in bacterial lysates removal of the linker region activated the ribosomal inactivating activity of the protein at least 250-fold, but that additional processing from the ends of the protein increased the activity.

Another genetic construction was made using PCR technology to remove the leader region from R34-DL. The new construction called R30-DL (nucleotides C-40 to C-84 and A-520 to A-594 inclusive are deleted) encoded a protein (approximately 29.5 kD) which was slightly smaller than R34-DL. *E.coli* lysates containing R30-DL appeared to be even more potent inhibitors of protein synthesis than R34-DL lysates. Papain treatment of R30-DL containing lysates further enhanced protein synthesis inhibiting activity. Following this treatment the R30-DL protein underwent a slight decrease in molecular weight representing processing at the ends of the polypeptide..

### Example 8:

A segment of the R30-DL gene was deleted which encodes several acidic residues at the carboxy terminus of the protein. The deletion was accomplished using the PCR engineering methods.

A thermocycler (Perkin-Elmer Cetus, Norwalk, CT) was used for the indicated constructions. A typical run was done with a one minute denaturation step, 2 minute annealing, and a 3 minute extension step. Temperatures used were 94°C, 37°C or 50°C, and 72°C respectively. Following 25 cycles the reaction was held at 72°C for 7 minutes for extension of unfinished products.

Amplification engineering reactions were done in four separate tubes of 100 µL each. The tubes were combined following amplification. Normally 100 ng of template was included in each tube. DNA primers were synthesized on a PCR Mate or 380A DNA synthesizer (Applied Biosystems) and were purified on acrylamide gels. Fifty (50) pmol of each primer were included in each reaction. The reaction conditions for the amplification were those recommended by Perkin Elmer Cetus (10 mM Tris-HCl pH8.3, 50 mM KCl, 1.5 mM MgCl2, 0.001 percent gelatin, 200 µM dNTPs and 2.5 units of *Taq* DNA polymerase or AmpliTaq™ thermostable DNA polymerase).

Several methods of genetic engineering were employed to produce the genetic derivatives described below. The standard methods of DNA purification, restriction enzyme digestion, agarose gel analysis, DNA fragment isolation, ligation and transformation were as described (see Molecular Cloning: A Laboratory Manual, *supra* and Current Protocols in Molecular Biology, (1987), *supra*.

Enzymes used for the engineering were from one of three manufacturers (Pharmacia LKB Biotechnology; Bethesda Research Labs; or New England Biolabs, Beverly, MA). Buffers and protocols used were provided by the manufacturer.

Using PCR introduced engineering, a modified RIP fragment is amplified from an RIP plasmid template, purified, then used to replace the unmodified region in the RIP gene. All fragment replacements were done in RIP genes already inserted into a pGEMEX expression plasmid.

A pGEMEX plasmid containing R30-DL, which had the 3' half of the gene removed by *Nco* I and *Stu* I digestion followed by gel purification was used as the template for PCR. The 3' half of the RIP gene was replaced with the PCR modified fragment described below.

A 3' PCR primer was synthesized which encoded the 7 amino acid deletion near the carboxy terminus and introduced a new unique *Bam* HI site. The 5' primer directed the deletion of the αβ linker and included a *Nco* I site. The sequences of these primers is given in Table 7. The primers were used to amplify a modified DNA fragment from a pGEMEX R34-DL template. The amplified fragment was phenol extracted and ethanol precipitated. The insert DNA was cut with *Nco* I and ligated into the pGEMEX-R30-DL vector.

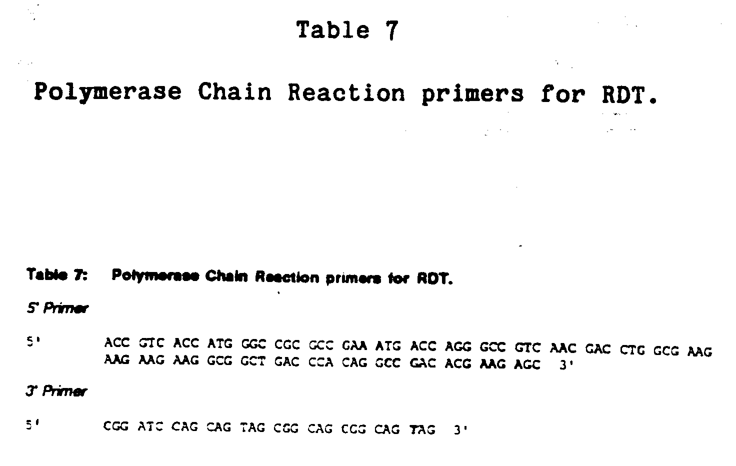

The new RIP gene derivative is called RDT and encodes a protein of predicted a 28,233 Daltons and pI ∼9.5. The RDT gene encodes a protein with a truncated leader, deleted linker and truncated carboxy terminus.

The DNA sequence for RDT is shown in Table 8.

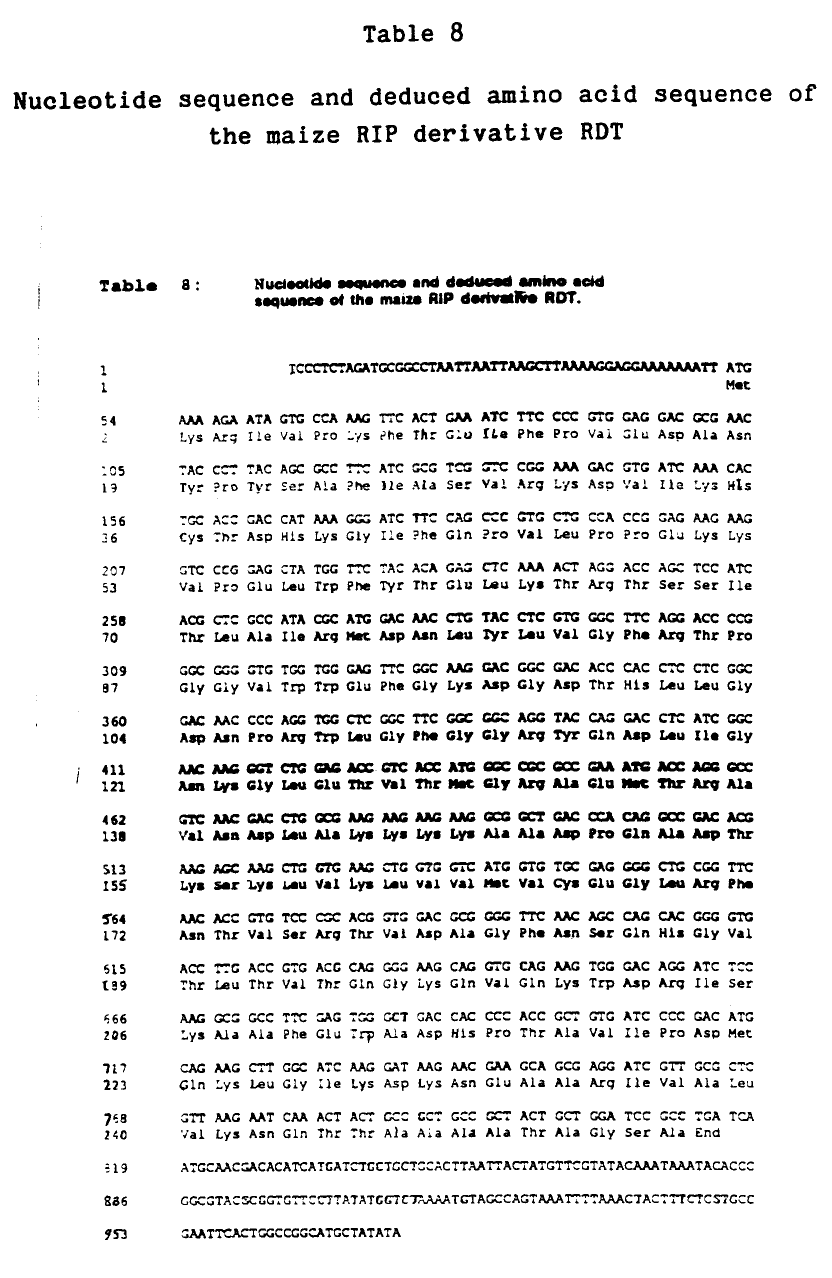

The RDT gene, expressed in *E.coli* using the pGEMEX system described above, was purified from bacterial lysates to apparent homogeneity. RDT protein appears to be a more potent inhibitor of protein synthesis than R30-DL. Using the reticulocyte lysate protein synthesis assay, purified RDT has a IC50 value of 1 ng/ml.

### Example 9:

RDT was further engineered to produce another gene called RDT-NP. This construction differs from RDT in having two unique restriction sites engineered into the gene. The sites were introduced using PCR methods described in Example 8. The PCR primer was designed such that it included the desired change and a unique restriction site in the maize RIP DNA sequence. A 99 bp primer which introduced the *Not* I and *Pst* I sites at the 3' end of the primer and had to be built back to the unique *Nco* I site for cloning purposes. The primers for the amplification are shown in Table 9.

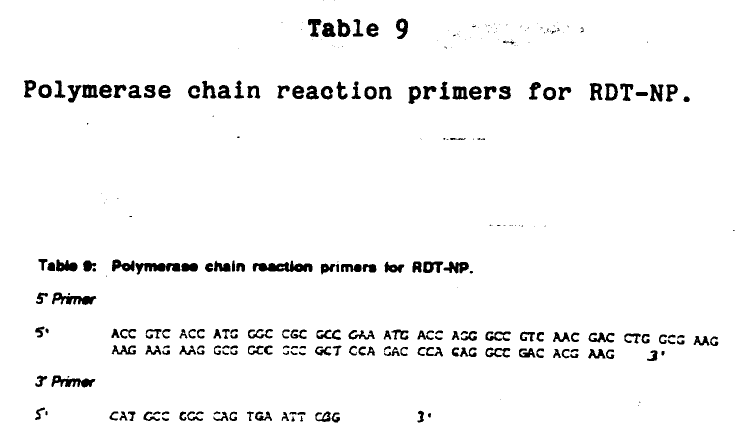

The restriction sites (*Not* I and *Pst* I) correspond to the site of the alpha/beta linker insertion in the RIP polypeptide. RDT-NP allows DNA segments encoding various polypeptide linkers to be inserted into the gene and tested for their ability to create an inactive, yet protease activatable RIP. The sequence for RDT-NP is shown in Table 10.

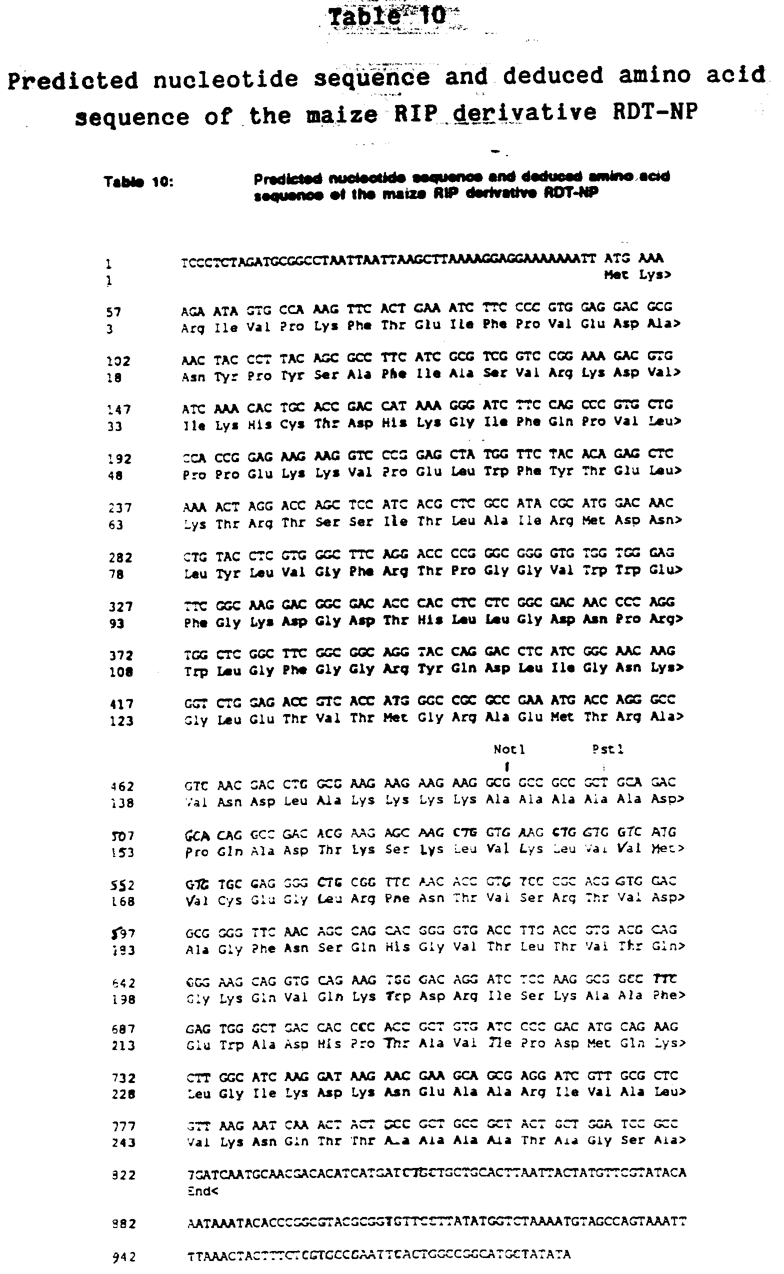

The RDT-NP polypeptide had a predicted molecular weight of 28,446 Daltons and pI of 9.5. Crude lysates of *E.coli* expressing RDT-NP from a pGEMEX vector are potent inhibitors of eukaryotic protein synthesis.

### Example 10:

To create an RIP molecule which would bind to immunoglobulin IgG, a single Antibody Binding Region (ABR) domain from the Staphylococcus aureus antibody binding Protein A was subcloned from the plasmid pRIT5 (Pharmacia LKB Biotechnology) using PCR techniques. The antibody binding domain of protein A (ABR-A) was PCR engineered to have a *Bam* HI site at its 5' end and a *Bgl* II site at it's 3' end. This allowed insertion of the ABR-A domain into the RDT *Bam* HI site while retaining the unique *Bam* HI site. The sequence of RDT-A is shown in Table 11.

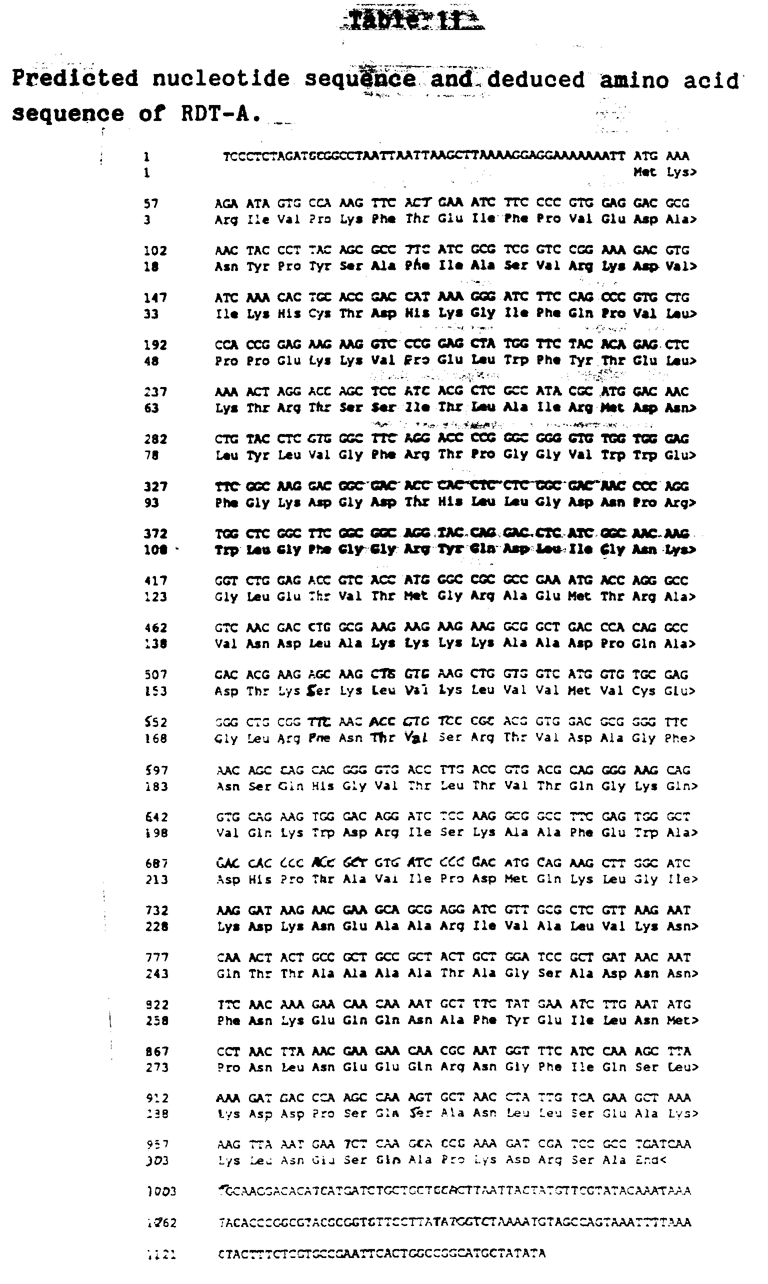

RDT-A was expressed in *E.coli* cells using the pGEMEX system. The resulting polypeptide had a predicted molecular weight of 35,198 Daltons and pI of 9.2. It was recognized by antisera to both protein A and maize RIP indicating the chimeric nature of the protein. Crude lysates of bacteria expressing RDT-A had potent eukaryotic protein synthesis inhibition activity.

RDT-A was shown to bind specifically to IgG Sepharose (Pharmacia LKB Biotechnology) following the manufacturer's instructions. Binding was best at pH 7.0. When washed at pH 5.0 the chimeric protein was released in small but detectable quantities from the resin. RDT alone does not bind to the gel.

### Example 11:

To increase the binding ability of the RDT-A to IgG antibodies the Antibody Binding Domain from Streptococcal Group G protein G (ABR-G) was synthesized using oligonucleotides. The sequence synthesized was that of the naturally occurring sequence described by Guss *et al.* ((1986), EMBO Journal, **5**:1567-1575). The only change was the addition of *Bam* HI and *Bgl* II sites at the 5' and 3' ends respectively of the synthetic DNA.

The ABR-G fragment was inserted into the *Bam* HI site of RDT-A. Two classes of clones have been studied. RDT-G-A contains a single ABR-G domain inserted in the correct orientation between the 3' end of RDT and the 5' end of ABR-A. A second class contains two properly oriented ABR-G domains. The predicted DNA sequences for the genes are shown in Tables 12 and 13.

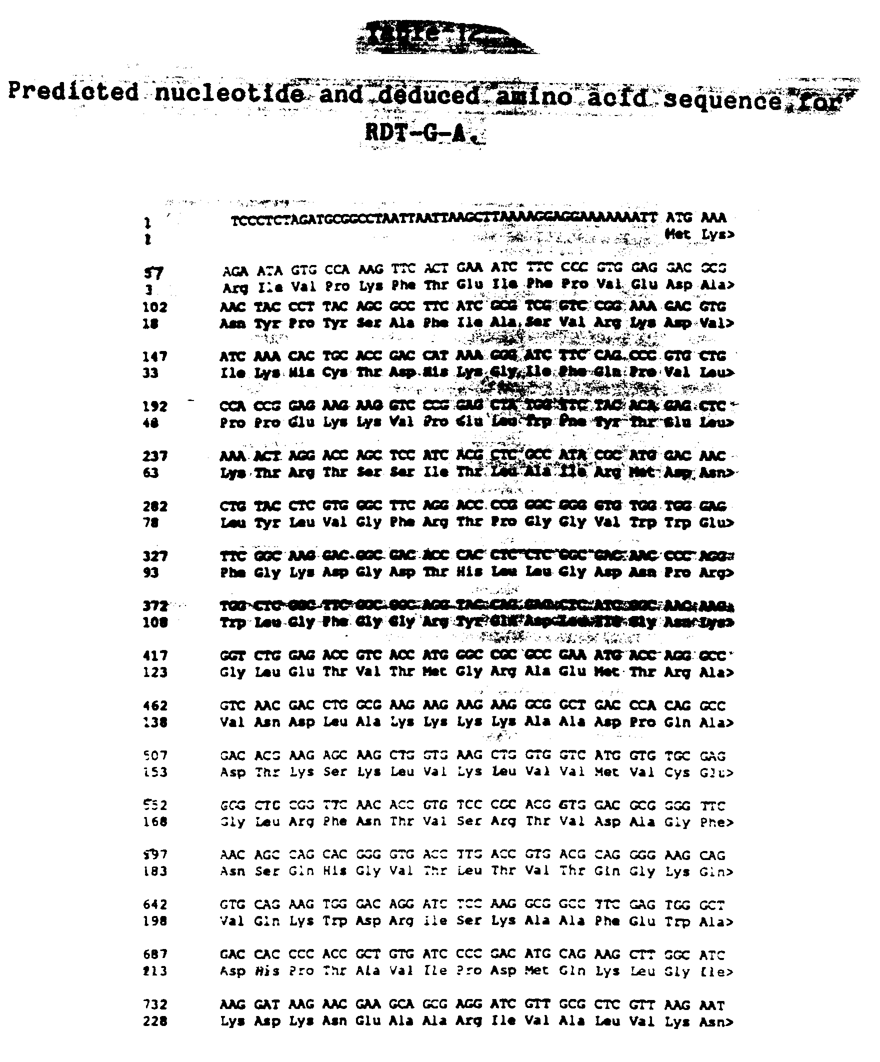

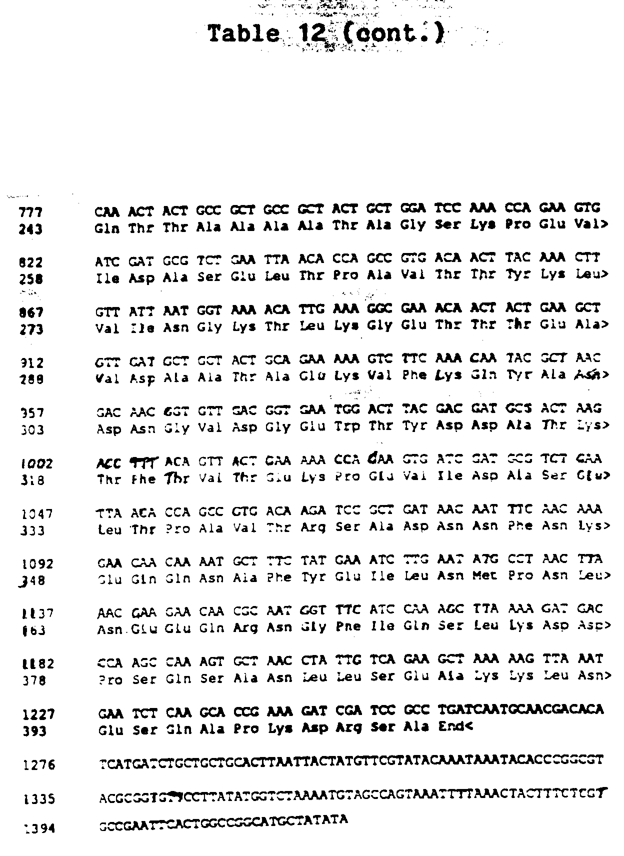

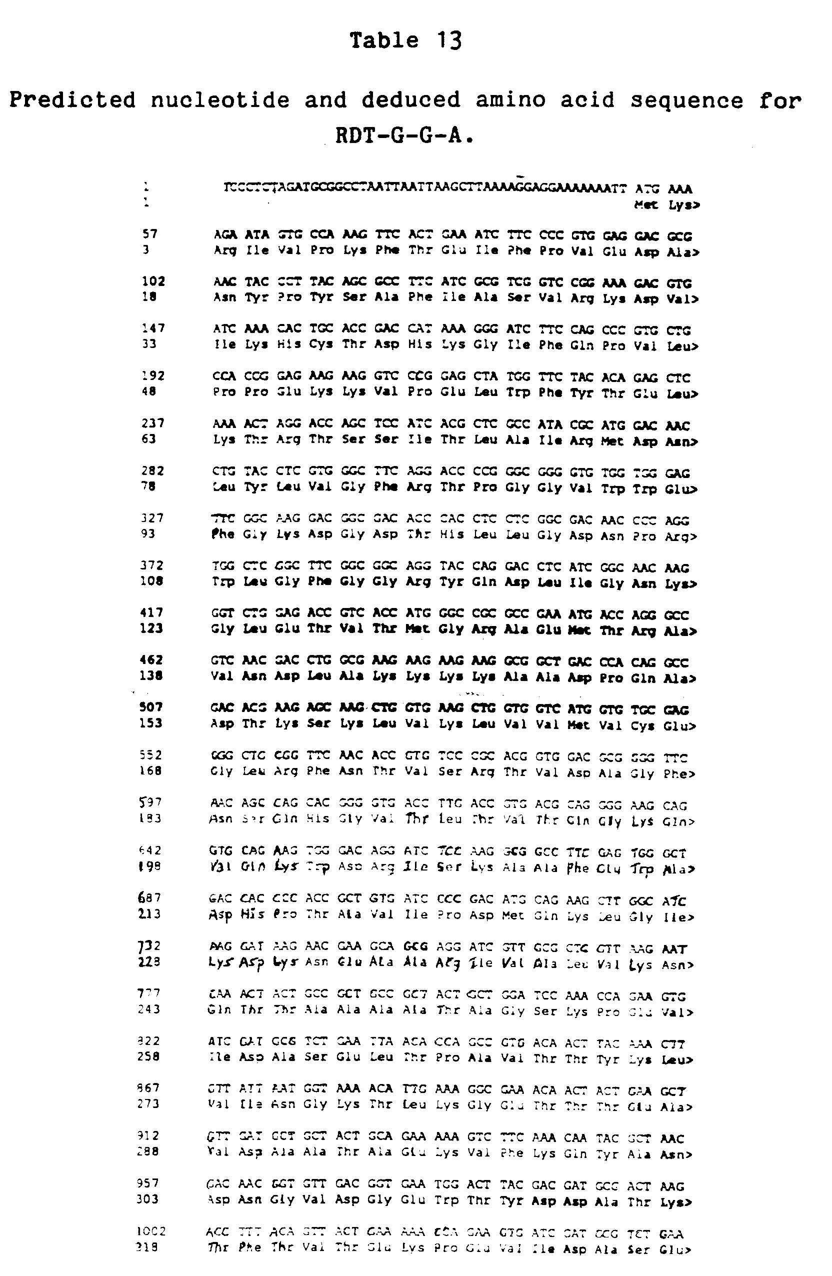

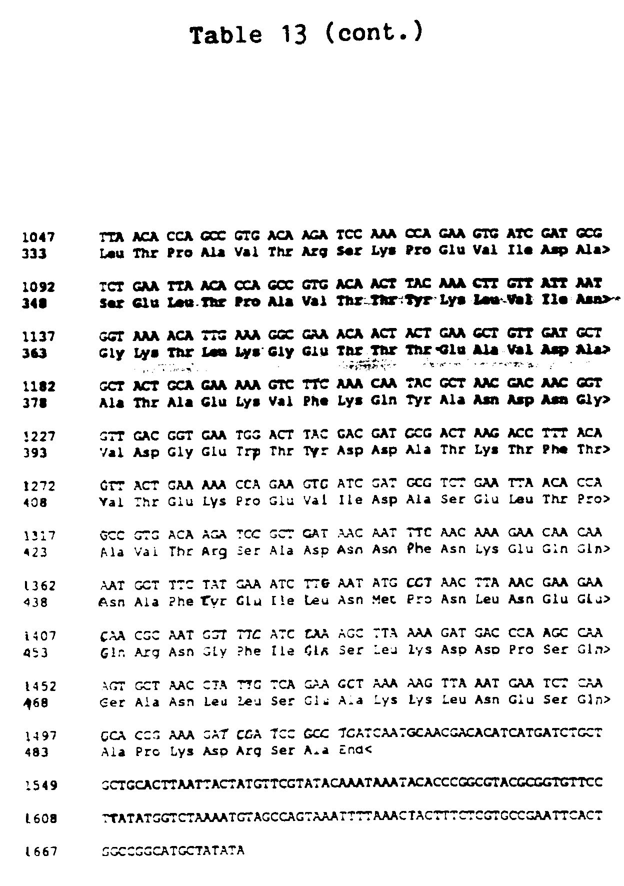

When these genes were expressed in *E.coli* using the pGEMEX system the expected chimeric proteins were produced. The RDT-G-A produced a protein of predicted molecular weight 44,576 Daltons (pI 7.2). RDT-G-G-A produced a slightly larger polypeptide predicted to be 53,955 Daltons with a predicted pI of 5.4.

Crude bacterial lysates of cells expressing RDT-G-A or RDT-G-G-A were potent inhibitors or eukaryotic protein synthesis in the rabbit reticulocyte assay described in Example 1. Papain treatment of the lysates further increases activity. Analysis of the papain treated lysates indicates that the intact RDT domain is released from the ABR domains.

Both RDT-G-A and RDT-G-G-A bind tightly to IgG Sepharose (Pharmacia LKB Biotechnology). Binding is stable at pH 5.0 Elution was accomplished with 0.5 M ammonium acetate pH 3.5 or by boiling the resin in SDS.

### Example 12:

Seeds from the following species of *Panicoideae* were ground to a fine consistency in a mortar and pestle (after removal of the glume if necessary): *Zea mays mays*, *Z.m. mexicana*, *Z.m. parviglumis*, *Z. luxurians*, *Z. mexicana*, *Z. mexicana*, *Tripsacum dactyloides*, *Coix lachryma-jobi*, *Sorghum bicolor*.

Soluble proteins were extracted from mature dry seed by the following techniques. The proteins were extracted for 2 hours with 50 mM sodium phosphate buffer, pH 7.5 containing 25 µg/ml leupeptin, 25 µg/ml antipain, 2 mM EDTA and 4 mM phenylmethane sulfonyl fluoride. Three milliliter extraction buffer per gram seed tissue was used. After centrifugation to remove insoluble material, an aliquot of the extract was analyzed by sodium dodecyl sulfate-polyacrylamide gel electrophoresis using 17-27% gradient gels, then electroblotted onto PVDF paper (Millipore). The blot was probed using rabbit antisera against maize RIP α and β fragments (1:2000 dilution each) as primary antibody and goat anti-rabbit IgG antibody as secondary antibody, then developed with NBT/BCIP.

Except the *Coix* extract, all the extracts tested showed cross-reactivity with the maize RIP antisera. A prominent band at ∼34 kD was observed corresponding to proRIP, and bands at ∼16.5 and 8.5 kD were also observed, corresponding to α and β fragments respectively. This indicates that RIP forms equivalent to maize proRIP and the activated αβ form are present in many members of the subfamily Panicoideae.

### Example 13:

Total DNA from the following species was isolated according to the procedures described by Saghai-Maroof et al., *PNAS*, **81**:8014-8018, 1984. The following species of *Panicoideae* were included: 3 accessions of *Zea mays* ssp.; *parviglumis*; *Zea luxurians*; *Zea mays ssp. mexicana*; *Coix lachryma-jobi*; *Sorghum bicolor*; and *Zea mays* ssp. *mays* var. B73.

Generally, about 8 µg of the extracted DNA from each sample was digested to completion with 20 units of *Hind* III, *Eco* RI and *Sst* I in 20 microliters of a reaction mixture containing the appropriate reaction buffer at 37°C for 2 hours.

Next, the total extracted DNA from each sample was subjected to the Southern hybridization technique (see Southern (1975), *J.Mol.Biol.*, **98**:503-517). The DNA fragments were fractionated on the basis of their size by means of electrophoresis on a 0.8% agarose gel. The double-stranded DNA fragments were modified into single-stranded DNA fragments in an alkali solution; and then a nylon filter was placed into close contact with the gel to transfer the modified DNA segments onto the filter in the presence of a high salt concentration solution.

Hybridization was carried out using, as the probe, the cDNA clone of the RIP gene (; the probe fragment is provided from position 2 to 1075 in Table 1). The probe was radiolabeled with ³²P and the signals in Southern transfers were visualized by autoradiography.

The Southern blots were hybridized with the cDNA clone of the RIP gene. A single fragment with strong homology to the clone was observed for each enzyme/species combination except the *Coix* accession. The inbred line of maize has a single major band with two minor bands. The other species which, with the exception of *Sorghum*, are not inbred showed between 2 and 5 minor bands. The *Coix* had either 4 or 5 such bands depending on the enzyme used.

## Claims

1. A DNA isolate encoding a maize proRIP having a ribosome inactivating protein sequence and a linker sequence wherein the maize proRIP has the following amino acid sequence:

1 KRIVPKFTEI FPVEDANYPY SAFIASVRKD VIKHCTDHKG
41 IFQPVLPPEK KVPELWFYTE LKTRTSSITL AIRMDNLYLV
81 GFRTPGGVWW EFGKDGDTHL LGDNPRWLGF GGRYQDLIGN
121 KGLETVTMGR AEMTRAVNDL AKKKKMATLE EEEVKMQMQM
161 PEAADLAAAA AADPQADTKS KLVKLVVMVC EGLRFNTVSR
201 TVDAGFNSQH GVTLTVTQGK QVQKWDRISK AAFEWADHPT
241 AVIPDMQKLG IKDKNEAARI VALVKNQTTA AAATAASADN
281 DDDEA

2. A DNA isolate encoding a protein capable of substantially inactivating eukaryotic ribosomes, said protein having the following amino acid sequence:

MAEITLEPSD LMAQTNKRIV PKFTEIFPVE DANYPYSAFI
ASVRKDVIKH CTDHKGIFQP VLPPEKKVPE LWFYTELKTR
TSSITLAIRM DNLYLVGFRT PGGVWWEFGK DGDTHLLGDN
PRWLGFGGRY QDLIGNKGLE TVTMGRAEMT RAVNDLAKKK
KAADPQADTK SKLVKLVVMV CEGLRFNTVS RTVDAGFNSQ
HGVTLTVTQG KQVQKWDRIS KAAFEWADHP TAVIPDMQKL
GIKDKNEAAR IVALVKNQTT AAAATAASAD NDDDEA

3. A biologically functional expression vehicle containing a DNA isolate of claims 1, 2, 7, 8, 9, or 10.

4. A host cell transformed with the biologically functional expression vehicle of claim 3.

5. The transformed host cell of claim 4, wherein the host cell is a eukaryotic cell.

6. The host cell of claim 5, wherein the host cell is maize.

7. A DNA isolate encoding a protein capable of substantially inactivating eukaryotic ribosomes, said protein having the following amino acid sequence:

MKRIVPKFTE IFPVEDANYP YSAFIASVRK DVIKHCTDHK
GIFQPVLPPE KKVPELWFYT ELKTRTSSIT LAIRMDNLYL
VGFRTPGGVW WEFGKDGDTH LLGDNPRWLG FGGRYQDLIG
NKGLETVTMG RAEMTRAVND LAKKKKAADP QADTKSKLVK
LVVMVCEGLR FNTVSRTVDA GFNSQHGVTL TVTQGKQVQK
WDRISKAAFE WADHPTAVIP DMQKLGIKDK NEAARIVALV
KNQTTAAAAT AASADNDDDE A

8. The DNA isolate of claim 1 designated R34 as shown in Table 6.

9. The DNA isolate of claim 2 designated R34-DL as shown in Example 7B.

10. The DNA isolate of claim 7 designated as R30-DL as shown in Example 7B.

## Patentansprüche

1. DNA-Isolat, kodierend für ein Mais-proRIP mit einer ribosomeninaktivierenden Proteinsequenz und einer Linkersequenz, wobei das Mais-proRIP die folgende Aminosäuresequenz aufweist:

1 KRIVPKFTEI FPVEDANYPY SAFIASVRKD VIKHCTDHKG
41 IFQPVLPPEK KVPELWFYTE LKTRTSSITL AIRMDNLYLV
81 GFRTPGGVWW EFGKDGDTHL LGDNPRWLGF GGRYQDLIGN
121 KGLETVTMGR AEMTRAVNDL AKKKKMATLE EEEVKMQMQM
161 PEAADLAAAA AADPQADTKS KLVKLVVMVC EGLRFNTVSR
201 TVDAGFNSQH GVTLTVTQGK QVQKWDRISK AAFEWADHPT
241 AVIPDMQKLG IKDKNEAARI VALVKNQTTA AAATAASADN
281 DDDEA

2. DNA-Isolat, kodierend für ein Protein, das in der Lage ist eukariotische Ribosomen wesentlich zu inaktivieren, wobei das Protein die folgende Aminosäuresequenz aufweist:

MAEITLEPSD LMAQTNKRIV PKFTEIFPVE DANYPYSAFI
ASVRKDVIKH CTDHKGIFQP VLPPEKKVPE LWFYTELKTR
TSSITLAIRM DNLYLVGFRT PGGVWWEFGK DGDTHLLGDN
PRWLGFGGRY QDLIGNKGLE TVTMGRAEMT RAVNDLAKKK
KAADPQADTK SKLVKLVVMV CEGLRFNTVS RTVDAGFNSQ
HGVTLTVTQG KQVQKWDRIS KAAFEWADHP TAVIPDMQKL
GIKDKNEAAR IVALVKNQTT AAAATAASAD NDDDEA

3. Biologisch funktionelles Expressionsvehikel, das ein DNA-Isolat nach Anspruch 1, 2, 7, 8, 9 oder 10 enthält.

4. Wirtszelle, die mit dem biologisch funktionellen Expressionsvehikel nach Anspruch 3 transformiert ist.

5. Transformierte Wirtszelle nach Anspruch 4, wobei die Wirtszelle eine eukariotische Zelle ist.

6. Wirtszelle nach Anspruch 5, wobei die Wirtszeile Mais ist.

7. DNA-Isolat, kodierend für ein Protein, das in der Lage ist eukariotische Ribosomen wesentlich zu inaktivieren, wobei das Protein die folgende Aminosäuresequenz aufweist:

MKRIVPKFTE IFPVEDANYP YSAFIASVRK DVIKHCTDHK
GIFQPVLPPE KKVPELWFYT ELKTRTSSIT LAIRMDNLYL
VGFRTPGGVW WEFGKDGDTH LLGDNPRWLG FGGRYQDLIG
NKGLETVTMG R[?]EMTRAVND LAKKKKAADP QADTKSKLVK
LVVMVCEGLR FNTVSRTVDA GFNSQHGVTL TVTQGKQVQK
WDRISKAAFE WADHPTAVIP DMQKLGIKDK NEAARIVALV
KNQTTAAAAT AASADNDDDE A

8. DNA-Isolat nach Anspruch 1, welches wie in Tabelle 6 gezeigt als R34 bezeichnet wird.

9. DNA-Isolat nach Anspruch 2, welches wie in Beispiel 7B gezeigt als R34-DL bezeichnet wird.

10. DNA-Isolat nach Anspruch 7, welches wie in Beispiel 7B gezeigt als R30-DL bezeichnet wird.

## Revendications

1. Isolat d'ADN codant une protéine proRIP de maïs qui comporte une séquence de protéine inactivant des ribosomes et une séquence de raccord, la protéine proRIP de maïs présentant la séquence suivante de résidus d'acides aminés :

1 KRIVPKFTEI FPVEDANYPY SAFIASVRKD VIKHCTDHKG
41 IFQPVLPPEK KVPELWFYTE LKTRTSSITL AIRMDNLYLV
81 GFRTPGGVWW EFGKDGDTHL LGDNPRWLGF GGRYQDLIGN
121 KGLETVTMGR AEMTRAVNDL AKKKKMATLE EEEVKMQMQM
161 PEAADLAAAA AADPQADTKS KLVKLVVMVC EGLRFNTVSR
201 TVDAGFNSQH GVTLTVTQGK QVQKWDRISK AAFEWADHPT
241 AVIPDMQKLG IKDKNEAARI VALVKNQTTA AAATAASADN
281 DDDEA

2. Isolat d'ADN codant une protéine capable de pratiquement inactiver des ribosomes de cellule eucaryote, cette protéine présentant la séquence suivante de résidus d'acides aminés :

MAEITLEPSD LMAQTNKRIV PKFTEIFPVE DANYPYSAFI
ASVRKDVIKH CTDHKGIFQP VLPPEKKVPE LWFYTELKTR
TSSITLAIRM DNLYLVGFRT PGGVWWEFGK DGDTHLLGDN
PRWLGFGGRY QDLIGNKGLE TVTMGRAEMT RAVNDLAKKK
KAADPQADTK SKLVKLVVMV CEGLRFNTVS RTVDAGFNSQ
HGVTLTVTQG KQVQKWDRIS KAAFEWADHP TAVIPDMQKL
GIKDKNEAAR IVALVKNQTT AAAATAASAD NDDDEA

3. Vecteur d'expression biologiquement fonctionnel, qui renferme un isolat d'ADN conforme à la revendication 1, 2, 7, 8, 9 ou 10.

4. Cellule hôte génétiquement modifiée au moyen d'un vecteur d'expression biologiquement fonctionnel, conforme à la revendication 3.

5. Cellule hôte génétiquement modifiée, conforme à la revendication 4, qui est une cellule eucaryote.

6. Cellule hôte conforme à la revendication 5, qui est une cellule de maïs.

7. Isolat d'ADN codant une protéine capable de pratiquement inactiver des ribosomes de cellule eucaryote, cette protéine présentant la séquence suivante de résidus d'acides aminés :

MKRIVPKFTE IFPVEDANYP YSAFIASVRK DVIKHCTDHK
GIFQPVLPPE KKVPELWFYT ELKTRTSSIT LAIRMDNLYL
VGFRTPGGVW WEFGKDGDTH LLGDNPRWLG FGGRYQDLIG
NKGLETVTMG RAEMTRAVND LAKKKKAADP QADTKSKLVK
LVVMVCEGLR FNTVSRTVDA GFNSQHGVTL TVTQGKQVQK
WDRISKAAFE WADHPTAVIP DMQKLGIKDK NEAARIVALV
KNQTTAAAAT AASADNDDDE A

8. Isolat d'ADN conforme à la revendication 1, appelé R34 et présenté dans le tableau 6.

9. Isolat d'ADN conforme à la revendication 2, appelé R34-DL et présenté dans l'exemple 7B.

10. Isolat d'ADN conforme à la revendication 7, appelé R30-DL et présenté dans l'exemple 7B.
